# EUROPÄISCHE PATENTSCHRIFT

(11) **EP 1 725 557 B1**
(45) Veröffentlichungstag und Bekanntmachung des Hinweises auf die Patenterteilung: **01.08.2007**
(21) Anmeldenummer: 05700961.5
(22) Anmeldetag: 15.01.2005
(51) Int. Cl.: C07D 487/04, A01N 43/90

(54) **6-(2-CHLOR-5-HALOGENPHENYL)-TRIAZOLOPYRIMIDINE, VERFAHREN ZU IHRER HERSTELLUNG UND IHRE VERWENDUNG ZUR BEKÄMPFUNG VON SCHADPILZEN SOWIE SIE ENTHALTENDE MITTEL**
6-(2-CHLORO-5-HALOPHENYL)-TRIAZOLOPYRIMIDINE, METHOD FOR PRODUCTION AND USE THEREOF FOR CONTROLLING FUNGAL PESTS AND AGENTS COMPRISING THE SAME
6-(2-CHLORO-5-HALOGENEPHENYL)-TRIAZOLOPYRIMIDINES, PROCEDE DE PRODUCTION DESDITS COMPOSES ET LEUR UTILISATION POUR LUTTER CONTRE LES CHAMPIGNONS PARASITES, ET AGENTS CONTENANT LESDITS COMPOSES

(30) Priorität: 23.01.2004 DE 102004003732; 19.10.2004 DE 102004051101
(43) Veröffentlichungstag der Anmeldung: 29.11.2006
(73) Patentinhaber: BASF Aktiengesellschaft, 67056 Ludwigshafen (DE)
(72) Erfinder: TORMO I BLASCO, Jordi, 69514 Laudenbach (DE); BLETTNER, Carsten, 68165 Mannheim (DE); MÜLLER, Bernd, 67227 Frankenthal (DE); GEWEHR, Markus, 56288 Kastellaun (DE); GRAMMENOS, Wassilios, 67071 Ludwigshafen (DE); GROTE, Thomas, 67157 Wachenheim (DE); RHEINHEIMER, Joachim, 67063 Ludwigshafen (DE); SCHÄFER, Peter, 67308 Ottersheim (DE); SCHIEWECK, Frank, 67258 Hessheim (DE); SCHWÖGLER, Anja, 68165 Mannheim (DE); WAGNER, Oliver, 67433 Neustadt (DE); SCHERER, Maria, 76829 Landau-Godramstein (DE); STRATHMANN, Siegfried, 67117 Limburgerhof (DE); SCHÖFL, Ulrich, 68782 Brühl (DE); STIERL, Reinhard, 67251 Freinsheim (DE)
(86) Internationale Anmeldenummer: PCT/EP2005/000377
(87) Internationale Veröffentlichungsnummer: WO 2005/070933

(56) Entgegenhaltungen:
- EP-A- 0 555 113
- WO-A-98/46608
- US-A- 4 567 263
- US-A- 5 994 360
- PATENT ABSTRACTS OF JAPAN Bd. 2003, Nr. 02, 5. Februar 2003 (2003-02-05) & JP 2002 308879 A (NIPPON SODA CO LTD), 23. Oktober 2002 (2002-10-23)

## Beschreibung

Die vorliegende Erfindung betrifft substituierte Triazolopyrimidine der Formel 1 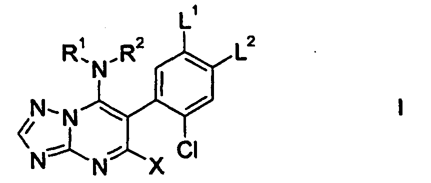 in der die Substituenten folgende Bedeutung haben:
- R¹, R²: unabhängig voneinander Wasserstoff, C₁-C₈-Alkyl, C₁-C₈-Halogenalkyl, C₃-C₈-Cycloalkyl, C₃-C₈-Halogencycloalkyl, C₂-C₈-Alkenyl, C₂C₈-Halogenalkenyl, C₃-C₆-Cycloalkenyl, C₃-C₈-Halogencycloalkenyl, C₂-C₈-Alkinyl, C₂-C₈-Halogenalkinyl oder Phenyl, Naphthyl, oder ein fünf- oder sechsgliedriger gesättigter, partiell ungesättigter oder aromatischer Heterocyclus, enthaltend ein bis vier Heteroatome aus der Gruppe O, N oder S,
R¹ und R² können auch zusammen mit dem Stickstoffatom, an das sie gebunden sind, ein fünf- oder sechsgliedriges Heterocyclyl oder Heteroaryl bilden, welches über N gebunden ist und ein bis drei weitere Heteroatome aus der Gruppe O, N und S als Ringglied enthalten und/oder einen oder mehrere Substituenten aus der Gruppe Halogen, C₁-C₆-Alkyl, C₁-C₆-Halogenalkyl, C₂-C₆-Alkenyl, C₂-C₆-Halogenalkenyl, C₁-C₆-Alkoxy, C₁-C₆-Halogenalkoxy, C₃-C₆-Alkenyloxy, C₃-C₆-Halogenalkenyloxy, C₁-C₆-Alkylen und Oxy-C₁-C₃-alkylenoxy tragen kann;
- R¹ und/oder R²: können eine bis vier gleiche oder verschiedene Gruppen R^{a} tragen:
R^{a} Halogen, Cyano, Nitro, Hydroxy, C₁-C₆-Alkyl, C₁-C₆-Halogenalkyl, C₁-C₆-Alkylcarbonyl, C₃-C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₂-C₈-Alkenyl, C₂-C₈-Halogenalkenyl, C₃-C₈-Cycloalkenyl, C₂-C₆-Alkenyloxy, C₃-C₆-Halogenalkenyloxy, C₂-C₆-Alkinyl, C₂-C₆-Halogenalkinyl, C₃-C₆-Alkinyloxy, C₃-C₆-Halogenalkinyloxy, C₃-C₆-Cycloalkoxy, C₃-C₆-Cycloalkenoxy, C₁-C₃-Qxyalkylenoxy, Phenyl, Naphthyl, fünf- bis zehngliedriger gesättigter, partiell ungesättigter oder aromatischer Heterocyclus, enthaltend ein bis vier Heteroatome aus der Gruppe O, N oder S, wobei diese aliphatischen, alicyclischen oder aromatischen Gruppen ihrerseits partiell oder vollständig halogeniert sein oder eine bis drei Gruppen R^{b} tragen können:
- R^{b}: Halogen, Cyano, Nitro, Hydroxy, Mercapto, Amino, Carboxyl, Aminocarbonyl, Aminothiocarbonyl, Alkyl, Haloalkyl, Alkenyl, Alkenyloxy, Alkinyloxy, Alkoxy, Halogenalkoxy, Alkylthio, Alkylamino, Dialkylamino, Formyl, Alkylcarbonyl, Alkylsulfonyl, Alkylsulfoxyl, Alkoxycarbonyl, Alkylcarbonyloxy, Alkylaminocarbonyl, Dialkylaminocarbonyl, Alkylaminothiocarbonyl, Dialkylaminothiocarbonyl, wobei die Alkylgruppen in diesen Resten 1 bis 6 Kohlenstoffatome enthalten und die genannten Alkenyl- oder Alkinylgruppen in diesen Resten 2 bis 8 Kohlenstoffatome enthalten;
und/oder einen bis drei der folgenden Reste:
Cycloalkyl, Cycloalkoxy, Heterocyclyl, Heterocyclyloxy, wobei die cyclischen Systeme 3 bis 10 Ringglieder enthalten; Aryl, Aryloxy, Arylthio, Aryl-C₁-C₆-alkoxy, Aryl-C₁-C₆-alkyl, Hetaryl, Hetaryloxy, Hetarylthio, wobei die Arylreste vorzugsweise 6 bis 10 Ringglieder, die Hetarylreste 5 oder 6 Ringglieder enthalten, wobei die cyclischen Systeme partiell oder vollständig halogeniert oder durch Alkyl- oder Haloalkylgruppen substituiert sein können;
- L¹: Fluor, Chlor oder Brom;
- L²: Wasserstoff, C₁-C₄-Alkyl oder C₁-C₄-Alkoxy; und
- X: Halogen, Cyano, C₁-C₄-Alkyl, C₁-C₄-Halogenalkyl, C₁-C₄-Alkoxy oder C₁-C₂-Halogenalkoxy.

Außerdem betrifft die Erfindung Verfahren und Zwischenprodukte zur Herstellung dieser Verbindungen, sie enthaltende Mittel sowie ihre Verwendung zur Bekämpfung von pflanzenpathogenen Schadpilzen.

Aus EP-A 71 792, EP-A 550 113 sind 5-Chlor-6-phenyl-7-amino-triazolopyrimidine allgemein bekannt. In EP-A 550 113, FR-A 27 84 991, US 5 994 360, WO 98/46608, JP-A 2002/308 879, WO 02/38565, WO 02/083677, WO 02/088125, WO 02/088126 und WO 02/088127 werden 6-(2-Cl-Phenyl)-7-amino-triazolopyrimidine allgemein vorgeschlagen, deren 6-Phenylgruppe eine weitere Halogensubstitution aufweist. Triazolopyrimidine, in denen die 6-Phenylgruppe 4-Alkyl- oder 4-Alkoxysubstituenten trägt, sind aus WO 98/48893, WO 03/008417 und WO 03/093271 bekannt. Diese Verbindungen sind zur Bekämpfung von Schadpilzen bekannt.

Die erfindungsgemäßen Verbindungen unterscheiden sich von den in den vorgenannten Schriften beschriebenen durch die 2,5-Disubstitution des 6-Phenylringes.

Die Wirkung der bekannten Verbindungen ist in vielen Fällen nicht zufriedenstellend. Davon ausgehend, liegt der vorliegenden Erfindung die Aufgabe zugrunde, Verbindungen mit verbesserter Wirkung und/oder verbreitertem Wirkungsspektrum bereitzustellen.

Demgemäss wurden die eingangs definierten Verbindungen gefunden. Des weiteren wurden Verfahren und Zwischenprodukte zu ihrer Herstellung, sie enthaltende Mittel sowie Verfahren zur Bekämpfung von Schadpilzen unter Verwendung der Verbindungen I gefunden.

Die erfindungsgemäßen Verbindungen können auf verschiedenen Wegen erhalten werden. Vorteilhaft werden sie durch Umsetzung von 5-Aminotriazol der Formel II mit entsprechend substituierten Phenylmalonaten der Formel III, in der R für Alkyl, bevorzugt für C₁-C₆-Alkyl, insbesondere für Methyl oder Ethyl steht, dargestellt.

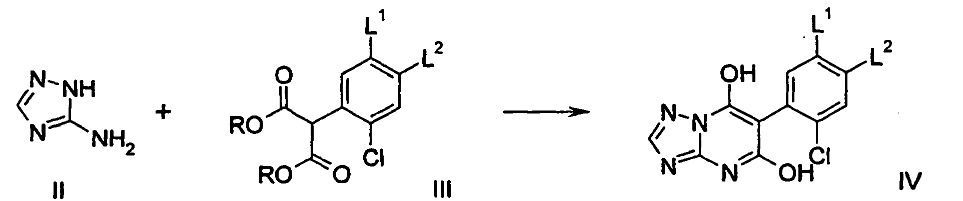

Diese Umsetzung erfolgt üblicherweise bei Temperaturen von 80°C bis 250°C, vorzugsweise 120°C bis 180°C, ohne Solvens oder in einem inerten organischen Lösungsmittel in Gegenwart einer Base [vgl. EP-A 770 615] oder in Gegenwart von Essigsäure unter den aus Adv. Het. Chem. Bd. 57, S. 81ff. (1993) bekannten Bedingungen.

Geeignete Lösungsmittel sind aliphatische Kohlenwasserstoffe, aromatische Kohlenwasserstoffe wie Toluol, o-, m- und p-Xylol, halogenierte Kohlenwasserstoffe, Ether, Nitrile, Ketone, Alkohole, sowie N-Methylpyrrolidon, Dimethylsulfoxid, Dimethylformamid und Dimethylacetamid. Besonders bevorzugt wird die Umsetzung ohne Lösungsmittel oder in Chlorbenzol, Xylol, Dimethylsulfoxid, N-Methylpyrrolidon durchgeführt. Es können auch Gemische der genannten Lösungsmittel verwendet werden.

Als Basen kommen allgemein anorganische Verbindungen wie Alkalimetall- und Erdalkalimetallhydroxide, Alkalimetall- und Erdalkalimetalloxide, Alkalimetall- und Erdalkalimetallhydride, Alkalimetallamide, Alkalimetall- und Erdalkalimetallcarbonate sowie Alkalimetallhydrogencarbonate, metallorganische Verbindungen, insbesondere Alkalimetallalkyle, Alkylmagnesiumhalogenide sowie Alkalimetall- und Erdalkalimetallalkoholate und Dimethoxymagnesium, außerdem organische Basen, z.B. tertiäre Amine wie Trimethylamin, Triethylamin, Tri-isopropylethylamin, Tributylamin und N-Methylpiperidin, N-Methylmorpholin, Pyridin, substituierte Pyridine wie Collidin, Lutidin und 4-Dimethylaminopyridin sowie bicyclische Amine in Betracht. Besonders bevorzugt werden tertiäre Amine wie Tri-isopropylethylamin, Tributylamin, N-Methylmorpholin oder N-Methylpiperidin.

Die Basen werden im allgemeinen in katalytischen Mengen eingesetzt, sie können aber auch äquimolar, im Überschuss oder gegebenenfalls als Lösungsmittel verwendet werden.

Die Edukte werden im allgemeinen in äquimolaren Mengen miteinander umgesetzt. Es kann für die Ausbeute vorteilhaft sein, die Base und das Malonat III in einem Überschuss bezogen auf das Triazol einzusetzen.

Phenylmalonate der Formel III werden vorteilhaft aus der Reaktion entsprechend substituierter Brombenzole mit Dialkylmalonaten unter Cu(l)-Katalyse erhalten [vgl. Chemistry Letters, S. 367-370, 1981; EP-A 10 02 788].

Die Dihydroxytriazolopyrimidine der Formel IV werden unter den aus WO-A 94/20501 bekannten Bedingungen in die Dihalogenpyrimidine der Formel V überführt, in der Y ein Halogenatom, bevorzugt ein Brom oder ein Chloratom, insbesondere ein Chloratom bedeutet. Als Halogenierungsmittel [HAL] wird vorteilhaft ein Chlorierungsmittel oder ein Bromierungsmittel, wie Phosphoroxybromid oder Phosphoroxychlorid, ggf. in Anwesenheit eines Lösungsmittels, eingesetzt.

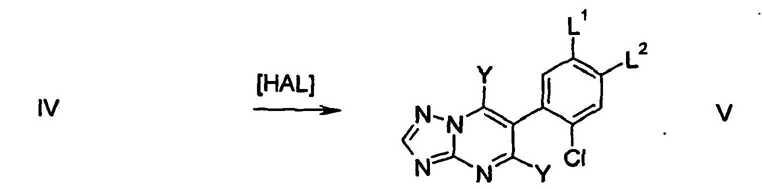

Diese Umsetzung wird üblicherweise bei 0°C bis 150°C, bevorzugt bei 80°C bis 125°C, durchgeführt [vgl. EP-A 770 615].

Dihalogenpyrimidine der Formel V werden mit Aminen der Formel VI, 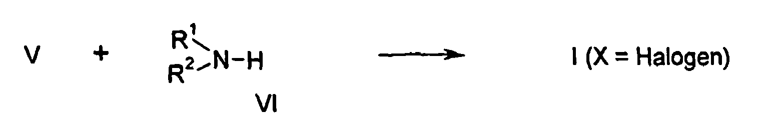 in der R¹ und R² wie in Formel I definiert sind, zu Verbindungen der Formel I, in der X für Halogen steht, weiter umgesetzt.

Diese Umsetzung wird vorteilhaft bei 0°C bis 70°C, bevorzugt 10°C bis 35°C durchgeführt, vorzugsweise in Anwesenheit eines inerten Lösungsmittels, wie Ether, z. B. Dioxan, Diethylether oder insbesondere Tetrahydrofuran, halogenierte Kohlenwasserstoffe, wie Dichlormethan und aromatische Kohlenwasserstoffe, wie beispielsweise Toluol [vgl. WO-A 98/46608].

Die Verwendung einer Base, wie tertiäre Amine, beispielsweise Triethylamin oder anorganische Basen, wie Kaliumcarbonat ist bevorzugt; auch überschüssiges Amin der Formel VI kann als Base dienen.

Verbindungen der Formel I, in der X Cyano, C₁-C₆-Alkoxy oder C₁-C₂-Halogenalkoxy bedeutet, können vorteilhaft aus der Umsetzung von Verbindungen 1, in der X Halogen, bevorzugt Chlor bedeutet, mit Verbindungen M-X' (Formel VII) erhalten werden. Verbindungen VII stellen je nach der Bedeutung der einzuführenden Gruppe X' ein anorganisches Cyanid, ein Alkoxylat oder ein Halogenalkoxylat dar. Die Umsetzung erfolgt vorteilhaft in Anwesenheit eines inerten Lösungsmittels. Das Kation M in Formel VII hat geringe Bedeutung; aus praktischen Gründen sind üblicherweise Ammonium-, Tetraalkylammonium- oder Alkali- oder Erdalkalimetallsalze bevorzugt.

I (X = Halogen) + M-X' → I (X = X') VII

Üblicherweise liegt die Reaktionstemperatur bei 0 bis 120°C, bevorzugt bei 10 bis 40°C [vgl. J. Heterocycl. Chem., Bd.12, S. 861-863 (1975)].

Geeignete Lösungsmittel umfassen Ether, wie Dioxan, Diethylether und, bevorzugt Tetrahydrofuran, Alkohole, wie Methanol oder Ethanol, halogenierte Kohlenwasserstoffe wie Dichlormethan und aromatische Kohlenwasserstoffe, wie Toluol oder Acetonitril.

Verbindungen der Formel l, in denen X für C₁-C₄-Alkyl oder C₁-C₄-Halogenalkyl steht, können vorteilhaft durch folgenden Syntheseweg erhalten werden:

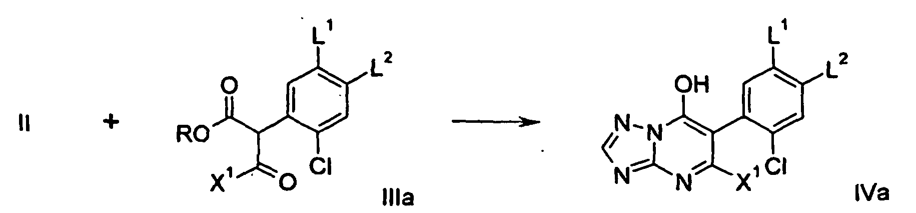

Ausgehend von den Ketoestem IIIa werden die 5-Alkyl-7-hydroxy-6-phenyltriazolopyrimidine IVa erhalten. In Formeln IIIa und IVa steht X¹ für C₁-C₄-Alkyl oder C₁-C₄-Halogenalkyl. Durch Verwendung der leicht zugänglichen 2-Phenylacetessigestem (IIIa mit X¹=CH₃) werden die 5-Methyl-7-hydroxy-6-phenyltriazolopyrimidine erhalten [vgl. Chem. Pharm. Bull., 9, 801, (1961)]. Die Herstellung der Ausgangsverbindungen IIIa erfolgt vorteilhaft unter den aus EP-A 10 02 788 beschrieben Bedingungen.

Die so erhaltenen 5-Alkyl-7-hydroxy-6-phenyltriazolopyrimidine werden mit Halogenierungsmitteln [HAL] unter den weiter oben beschriebenen Bedingungen zu den 7-Halogenotriazolopyrimidinen der Formel Va umgesetzt, in der Y für ein Halogenatom steht. Bevorzugt werden Chlorierungs- oder Bromierungsmittel wie Phosphoroxybromid, Phosphoroxychlorid, Thionylchlorid, Thionylbromid oder Sulfurylchlorid eingesetzt. Die Umsetzung kann in Substanz oder in Gegenwart eines Lösungsmittels durchgeführt werden. Übliche Reaktionstemperaturen betragen von 0 bis 150°C oder vorzugsweise von 80 bis 125°C.

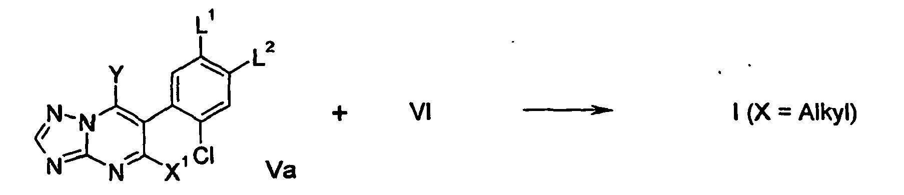

Die Umsetzung von Va mit Aminen VI erfolgt unter den weiter oben beschriebenen Bedingungen.

Verbindungen der Formel I in der X C₁-C₄-Alkyl bedeutet, können alternativ auch aus Verbindungen I, in der X Halogen, insbesondere Chlor, bedeutet und Malonaten der Formel VIII hergestellt werden. In Formel VIII bedeuten X" Wasserstoff oder C₁-C₃-Alkyl und R C₁-C₄-Alkyl. Sie werden zu Verbindungen der Formel IX umgesetzt und zu Verbindungen I decarboxyliert [vgl. US 5,994,360].

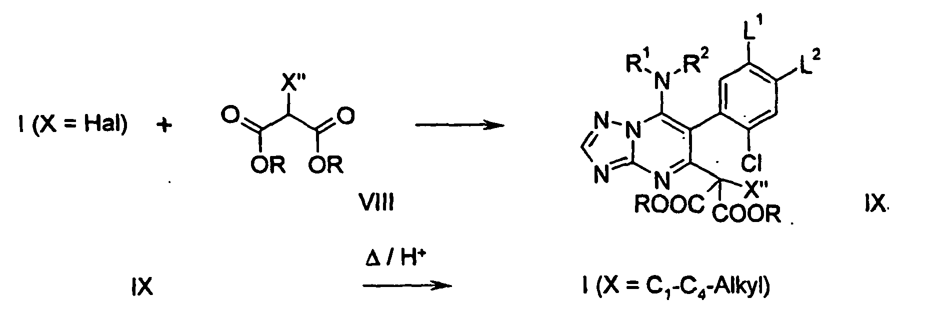

Die Malonate VIII sind in der Literatur bekannt [J. Am. Chem. Soc., Bd. 64, 2714 (1942); J. Org. Chem., Bd. 39, 2172 (1974); Helv. Chim. Acta, Bd. 61, 1565 (1978)] oder können gemäß der zitierten Literatur hergestellt werden.

Die anschließende Verseifung des Esters IX erfolgt unter allgemein üblichen Bedingungen, in Abhängigkeit der verschiedenen Strukturelemente kann die alkalische oder die saure Verseifung der Verbindungen IX vorteilhaft sein. Unter den Bedingungen der Esterverseifung kann die Decarboxylierung zu I bereits ganz oder teilweise erfolgen.

Die Decarboxylierung erfolgt üblicherweise bei Temperaturen von 20°C bis 180°C, vorzugsweise 50°C bis 120°C, in einem inerten Lösungsmittel, gegebenenfalls in Gegenwart einer Säure.

Geeignete Säuren sind Salzsäure, Schwefelsäure, Phosphorsäure, Ameisensäure, Essigsäure, p-Toluolsulfonsäure. Geeignete Lösungsmittel sind Wasser, aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert.-Butanol, sowie Dimethylsulfoxid, Dimethylformamid und Dimethylacetamid, besonders bevorzugt wird die Reaktion in Salzsäure oder Essigsäure durchgeführt. Es können auch Gemische der genannten Lösungsmittel verwendet werden.

Verbindungen der Formel I, in denen X für C₁-C₄-Alkyl steht, können auch durch Kupplung von 5-Halogentriazolopyrimidinen der Formel I, in der X Halogen bedeutet, mit metallorganischen Reagenzien der Formel X erhalten werden. In einer Ausführungsform dieses Verfahrens erfolgt die Umsetzung unter Übergangsmetallkatalyse, wie Nioder Pd-Katalyse.

I (X = Hal) + M^{y}(-X")_{y} → I (X = C₁-C₄-Alkyl) X

In Formel X steht M für ein Metallion der Wertigkeit Y, wie beispielsweise B, Zn oder Sn und X" für C₁-C₃-Alkyl. Diese Reaktion kann beispielsweise analog folgender Methoden durchgeführt werden: J. Chem. Soc. Perkin Trans. 1, 1187 (1994), ebenda 1, 2345 (1996); WO-A 99/41255; Aust. J. Chem., Bd. 43, 733 (1990); J. Org. Chem., Bd. 43, 358 (1978); J. Chem. Soc. Chem. Commun. 866 (1979); Tetrahedron Lett., Bd. 34, 8267 (1993); ebenda, Bd. 33, 413 (1992).

Die Reaktionsgemische werden in üblicher Weise aufgearbeitet, z.B. durch Mischen mit Wasser, Trennung der Phasen und gegebenenfalls chromatographische Reinigung der Rohprodukte. Die Zwischen- und Endprodukte fallen z.T. in Form farbloser oder schwach bräunlicher, zäher Öle an, die unter vermindertem Druck und bei mäßig erhöhter Temperatur von flüchtigen Anteilen befreit oder gereinigt werden. Sofern die Zwischen- und Endprodukte als Feststoffe erhalten werden, kann die Reinigung auch durch Umkristallisieren oder Digerieren erfolgen.

Sofern einzelne Verbindungen I nicht auf den voranstehend beschriebenen Wegen zugänglich sind, können sie durch Derivatisierung anderer Verbindungen I hergestellt werden.

Sofern bei der Synthese Isomerengemische anfallen, ist im allgemeinen jedoch eine Trennung nicht unbedingt erforderlich, da sich die einzelnen Isomere teilweise während der Aufbereitung für die Anwendung oder bei der Anwendung (z.B. unter Licht-, Säure- oder Baseneinwirkung) ineinander umwandeln können. Entsprechende Umwandlungen können auch nach der Anwendung, beispielsweise bei der Behandlung von Pflanzen in der behandelten Pflanze oder im zu bekämpfenden Schadpilz erfolgen.

Bei den in den vorstehenden Formeln angegebenen Definitionen der Symbole wurden Sammelbegriffe verwendet, die allgemein repräsentativ für die folgenden Substituenten stehen:
Halogen: Fluor, Chlor, Brom und Jod;
Alkyl: gesättigte, geradkettige oder verzweigte Kohlenwasserstoffreste mit 1 bis 4, 6 oder 8 Kohlenstoffatomen, z.B. C₁-C₆-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2- Trimethylpropyl, 1,2,2-Trimethylpropyl, 1-Ethyl-1-methylpropyl und 1-Ethyl-2-methylpropyl;
Halogenalkyl: geradkettige oder verzweigte Alkylgruppen mit 1 bis 2, 4 oder 6 Kohlenstoffatomen (wie vorstehend genannt), wobei in diesen Gruppen teilweise oder vollständig die Wasserstoffatome durch Halogenatome wie vorstehend genannt ersetzt sein können: insbesondere C₁-C₂-Halogenalkyl wie Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl, 2-Fluor ethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl oder 1,1,1-Trifluorprop-2-yl;
Alkenyl: ungesättigte, geradkettige oder verzweigte Kohlenwasserstoffreste mit 2 bis 4, 6 oder 8 Kohlenstoffatomen und einer oder zwei Doppelbindungen in beliebiger Position, z.B. C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyt-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2 Trimethyl-2-propenyl, 1-Ethyl-1-methyl-2-propenyl, 1-Ethyl-2-methyl-1-propenyl und 1-Ethyl-2-methyl-2-propenyl;
Halogenalkenyl: ungesättigte, geradkettige oder verzweigte Kohlenwasserstoffreste mit 2 bis 8 Kohlenstoffatomen und einer oder zwei Doppelbindungen in beliebiger Position (wie vorstehend genannt), wobei in diesen Gruppen die Wasserstoffatome teilweise oder vollständig gegen Halogenatome wie vorstehend genannt, insbesondere Fluor, Chlor und Brom, ersetzt sein können;
Alkinyl: geradkettige oder verzweigte Kohlenwasserstoffgruppen mit 2 bis 4, 6 oder 8 Kohlenstoffatomen und einer oder zwei Dreifachbindungen in beliebiger Position, z.B. C₂-C₆-Alkinyl wie Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl;
Cycloalkyl: mono- oder bicyclische, gesättigte Kohlenwasserstoffgruppen mit 3 bis 6 oder 8 Kohlenstoffringgliedern, z.B. C₃-C₈-Cycloalkyl wie Cyclopropyl, Cyclobutyl, Cyclopentyl, Cyclohexyl, Cycloheptyl und Cyclooctyl;
fünf- bis zehngliedriger gesättigter, partiell ungesättigter oder aromatischer Heterocyclus, enthaltend ein bis vier Heteroatome aus der Gruppe O, N oder S:
- 5- oder 6-gliedriges Heterocyclyl, enthaltend ein bis drei Stickstoffatome und/oder ein Sauerstoff- oder Schwefelatom oder ein oder zwei Sauerstoff- und/oder Schwefelatome, z.B. 2-Tetrahydrofuranyl, 3-Tetrahydrofuranyl, 2-Tetrahydrothienyl, 3-Tetrahydrothienyl, 2-Pyrrolidinyl, 3-Pyrrolidinyl, 3-Isoxazolidinyl, 4-Isoxazolidinyl, 5-Isoxazolidinyl, 3-lsothiazolidinyl, 4-Isothiazolidinyl, 5-lsothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl, 2-Pyrrolin-2-yl, 2-Pyrrolin-3-yl, 3-Pyrrolin-2-yl, 3-Pyrrolin-3-yl, 2-Piperidinyl, 3-Piperidinyl, 4-Piperidinyl, 1,3-Dioxan-5-yl, 2-Tetrahydropyranyl, 4-Tetrahydropyranyl, 2-Tetrahydrothienyl, 3-Hexahydropyridazinyl, 4-Hexahydropyridazinyl, 2-Hexahydropyrimidinyl, 4-Hexahydropyrimidinyl, 5-Hexahydropyrimidinyl und 2-Piperazinyl;
- 5-gliedriges Heteroaryl, enthaltend ein bis vier Stickstoffatome oder ein bis drei Stickstoffatome und ein Schwefel- oder Sauerstoffatom: 5-Ring Heteroarylgruppen, welche neben Kohlenstoffatomen ein bis vier Stickstoffatome oder ein bis drei Stickstoffatome und ein Schwefel- oder Sauerstoffatom als Ringglieder enthalten können, z.B. 2-Furyl, 3-Furyl, 2-Thienyl, 3-Thienyl, 2-Pyrrolyl, 3-Pyrrolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, und 1,3,4-Triazol-2-yl;
- 6-gliedriges Heteroaryl, enthaltend ein bis drei bzw. ein bis vier Stickstoffatome: 6-Ring Heteroarylgruppen, welche neben Kohlenstoffatomen ein bis drei bzw. ein bis vier Stickstoffatome als Ringglieder enthalten können, z.B. 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl und 2-Pyrazinyl;
Alkylen: divalente unverzweigte Ketten aus 3 bis 5 CH₂-Gruppen, z.B. CH₂, CH₂CH₂, CH₂CH₂CH₂, CH₂CH₂CH₂CH₂ und CH₂CH₂CH₂CH₂CH₂;
Oxyalkylen: divalente unverzweigte Ketten aus 2 bis 4 CH₂-Gruppen, wobei eine Valenz über ein Sauerstoffatom an das Gerüst gebunden ist, z.B. OCH₂CH₂, OCH₂CH₂CH₂ und OCH₂CH₂CH₂CH₂;
Oxyalkylenoxy: divalente unverzweigte Ketten aus 1 bis 3 CH₂-Gruppen, wobei beide Valenzen über ein Sauerstoffatom an das Gerüst gebunden ist, z.B. OCH₂O, OCH₂CH₂O und OCH₂CH₂CH₂O;

In dem Umfang der vorliegenden Erfindung sind die (R)- und (S)-Isomere und die Razemate von Verbindungen der Formel 1 eingeschlossen, die chirale Zentren aufweisen.

Die besonders bevorzugten Ausführungsformen der Zwischenprodukte in Bezug auf die Variablen entsprechen denen der Gruppen L der Formel I.

Im Hinblick auf ihre bestimmungsgemäße Verwendung der Triazolopyrimidine der Formel I sind die folgenden Bedeutungen der Substituenten, und zwar jeweils für sich allein oder in Kombination, besonders bevorzugt:

Verbindungen der Formel I werden bevorzugt, in denen R¹ nicht Wasserstoff bedeutet.

Verbindungen I werden besonders bevorzugt, in denen R¹ für C₁-C₆-Alkyl, C₂-C₆-Alkenyl, C₂-C₆-Alkinyl oder C₁-C₈-Halogenalkyl steht.

Verbindungen I sind bevorzugt, in denen R¹ für eine Gruppe A steht: 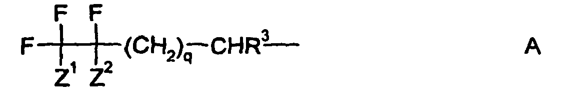 worin
- Z¹: Wasserstoff, Fluor oder C₁-C₆-Fluoroalkyl,
- Z²: Wasserstoff oder Fluor, oder
Z¹ und Z² bilden gemeinsam eine Doppelbindung;
- q: 0 oder 1 ist; und
- R³: Wasserstoff oder Methyl bedeuten.

Außerdem werden Verbindungen l bevorzugt, in denen R¹ für C₃-C₆-Cycloalkyl steht, welches durch C₁-C₄-Alkyl substituiert sein kann.

Insbesondere werden Verbindungen l bevorzugt, in denen R² Wasserstoff bedeutet.

Gleichermaßen bevorzugt sind Verbindungen l, in denen R² für Methyl oder Ethyl steht.

Sofern R¹ und/oder R² Halogenalkyl oder Halogenalkenylgruppen mit Chiralitätszentrum beinhalten, sind für diese Gruppen die (S)- lsomere bevorzugt. Im Fall halogenfreier Alkyl oder Alkenylgruppen mit Chiralitätszentrum in R¹ oder R² sind die (R)-konfigurierten Isomere bevorzugt.

Weiterhin werden Verbindungen l bevorzugt, in denen R¹ und R² zusammen mit dem Stickstoffatom, an das sie gebunden sind, einen Piperidinyl-, Morpholinyl- oder Thiomorpholinylring bilden, insbesondere einen Piperidinylring, der ggf. durch eine bis drei Gruppen Halogen, C₁-C₄-Alkyl oder C₁-C₄-Halogenalkyl substituiert ist. Besonders bevorzugt sind die Verbindungen, in denen R¹ und R² zusammen mit dem Stickstoffatom, an das sie gebunden sind, einen 4-Methylpiperidinring bilden.

Ein weiterer bevorzugter Gegenstand der Erfindung sind Verbindungen I, in denen R¹ und R² zusammen mit dem Stickstoffatom, an das sie gebunden sind, einen Pyrazolring bilden, der ggf. durch eine oder zwei Gruppen Halogen, C₁-C₄-Alkyl oder C₁-C₄-Halogenalkyl, insbesondere durch 3,5-Dimethyl oder 3,5-Di-(trifluorrnethyl) substituiert ist.

Daneben sind auch Verbindungen der Formel I besonders bevorzugt, in denen R¹ CH(CH₃)-CH₂CH₃ , CH(CH₃)-CH(CH₃)₂ , CH(CH₃)-C(CH₃)₃ , CH(CH₃)-CF₃ , CH₂C(CH₃)=CH₂ ,CH₂CH=CH₂, Cyclopentyl oder Cyclohexyl; R² Wasserstoff oder Methyl; oder R¹ und R² gemeinsam -(CH₂)₂CH(CH₃)(CH₂)₂-, -(CH₂)₂CH(CF₃)(CH₂)₂- oder -(CH₂)₂O(CH₂)₂- bedeuten.

Verbindungen I werden bevorzugt, in denen X Halogen, C₁-C₄-Alkyl, Cyano oder C₁-C₄-Alkoxy, wie Chlor, Methyl, Cyano, Methoxy oder Ethoxy, besonders Chlor oder Methyl, insbesondere Chlor bedeutet.

Ein bevorzugter Gegenstand der vorliegenden Erfindung sind Verbindungen I, in denen L¹ Chlor bedeutet.

Ein weiterer bevorzugter Gegenstand der vorliegenden Erfindung sind Verbindungen I, in denen L¹ Fluor bedeutet.

In einer anderen Ausgestaltung der vorliegenden Erfindung steht L¹ für Brom.

Ein besonders bevorzugter Gegenstand der vorliegenden Erfindung sind Verbindungen I, in denen L² Wasserstoff bedeutet.

In einer anderen Ausgestaltung der vorliegenden Erfindung steht L² für Methyl oder Methoxy.

Eine bevorzugte Ausführungsform der Erfindung betrifft Verbindungen der Formel I. 1: 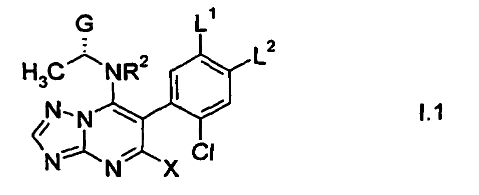 in der
- G: C₂-C₆-Alkyl, insbesondere Ethyl, n- und i-Propyl, n-, sek-, tert*-* Butyl, und C₁-C₄-Alkoxymethyl, insbesondere Ethoxymethyl, oder C₃-C₈-Cycloalkyl, insbesondere Cyclopentyl oder Cyclohexyl;
- R²: Wasserstoff oder Methyl;
- X: Chlor, Methyl, Cyano, Methoxy oder Ethoxy bedeuten und
- L¹ und L²: die Bedeutung gemäß Formel l aufweisen.

Eine weitere bevorzugte Ausführungsform der Erfindung betrifft Verbindungen, in denen R¹ und R² zusammen mit dem Stickstoffatom, an das sie gebunden sind, ein fünf- oder sechsgliedriges Heterocyclyl oder Heteroaryl bilden, welches über N gebunden ist und ein weiteres Heteroatom aus der Gruppe O, N und S als Ringglied enthalten und/oder einen oder mehrere Substituenten aus der Gruppe Halogen, C₁-C₆-Alkyl, C₁-C₆-Halogenalkyl, C₂-C₆-Alkenyl, C₂-C₆-Halogenalkenyl, C₁-C₆-Alkoxy, C₁-C₆-Halogenalkoxy, C₃-C₆-Alkenyloxy, C₃-C₆-Halogenalkenyloxy, C₁-C₆-Alkylen und Oxy-C₁-C₃-alkylenoxy tragen kann. Diese Verbindungen entsprechen insbesondere Formel 1.2, 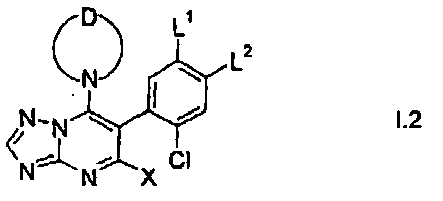 in der
- D: zusammen mit dem Stickstoffatom ein fünf- oder sechsgliedriges Heterocyclyl oder Heteroaryl bildet, welches über N gebunden ist und ein weiteres Hetero-atom aus der Gruppe O, N und S als Ringglied enthalten und/oder einen oder mehrere Substituenten aus der Gruppe Halogen, C₁-C₄-Alkyl, C₁-C₄-Alkoxy und C₁-C₂-Halogenalkyl tragen kann; und
- X: Chlor, Methyl, Cyano, Methoxy oder Ethoxy bedeuten und
- L¹ und L²: die Bedeutung gemäß Formel I aufweisen.

Eine weitere bevorzugte Ausführungsform der Erfindung betrifft Verbindungen der Formel I.3.

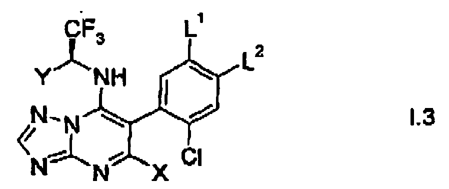

in der Y für Wasserstoff oder C₁-C₄-Alkyl, insbesondere für Methyl und Ethyl, X für Chlor, Methyl, Cyano, Methoxy oder Ethoxy steht und L¹ und L² die Bedeutung gemäß Formel 1 aufweisen.

Eine weitere bevorzugte Ausgestaltung der Erfindung betrifft Verbindungen; in denen L¹ Fluor und L² Alkyl oder Alkoxy, insbesondere Methyl oder Methoxy bedeuten.

Eine weitere bevorzugte Ausgestaltung der Erfindung betrifft Verbindungen, in denen L¹ Chlor und L² Alkyl oder Alkoxy, insbesondere Methyl oder Methoxy bedeuten.

Die Verbindungen I eignen sich als Fungizide. Sie zeichnen sich aus durch eine hervorragende Wirksamkeit gegen ein breites Spektrum von pflanzenpathogenen Pilzen, insbesondere aus der Klasse der *Ascomyceten, Deuteromyceten, Oomyceten* und *Basidiomyceten.* Sie sind zum Teil systemisch wirksam und können im Pflanzenschutz als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Bananen, Baumwolle, Soja, Kaffee, Zuckerrohr, Wein, Obst- und Zierpflanzen und Gemüsepflanzen wie Gurken, Bohnen, Tomaten, Kartoffeln und Kurbisgewächsen, sowie an den Samen dieser Pflanzen.

Speziell eignen sie sich zur Bekämpfung folgender Pflanzenkrankheiten:
- *Altemaria-Arten* an Gemüse und Obst,
- *Bipolaris-* und Drechslera-Arten an Getreide, Reis und Rasen,
- *Blumeria graminis* (echter Mehltau) an Getreide,
- *Botrytis cinerea* (Grauschimmel) an Erdbeeren, Gemüse, Zierpflanzen und Reben,
- *Erysiphe cichoracearum* und *Sphaerotheca fuliginea* an Kürbisgewächsen,
- *Fusarium-* und *Verticillium-Arten* an verschiedenen Pflanzen,
- Mycosphaerella-Arten an Getreide, Bananen und Erdnüssen,
- *Phytophthora infestans* an Kartoffeln und Tomaten,
- *Plasmopara viticola* an Reben,
- *Podosphaera leucotricha* an Äpfeln,
- *Pseudocercosporella herpatrichoides* an Weizen und Gerste,
- *PseudoperonosporarArten* an Hopfen und Gurken,
- *Puccinia-Arten* an Getreide,
- *Pyricularia* oryzae an Reis,
- Rhizoctonia-Arten an Baumwolle, Reis und Rasen,
- *Septoria tritici* und *Stagonospora nodorum* an Weizen,
- *Uncinula* necatoran Reben,
- Ustilago-Arten an Getreide und Zuckerrohr, sowie
- Venturia-Arten (Schorf) an Äpfeln und Birnen.

Die Verbindungen I eignen sich außerdem zur Bekämpfung von Schadpilzen wie Pae*cilomyces variotii* im Materialschutz (z.B. Holz, Papier, Dispersionen für den Anstrich, Fasern bzw. Gewebe) und im Vorratsschutz.

Die Verbindungen I werden angewendet, indem man die Pilze oder die vor Pilzbefall zu schützenden Pflanzen, Saatgüter, Materialien oder den Erdboden mit einer fungizid wirksamen Menge der Wirkstoffe behandelt. Die Anwendung kann sowohl vor als auch nach der Infektion der Materialien; Pflanzen oder Samen durch die Pilze erfolgen.

Die fungiziden Mittel enthalten im allgemeinen zwischen 0,1 und 95, vorzugsweise zwischen 0,5 und 90 Gew.-% Wirkstoff.

Die Aufwandmengen liegen bei der Anwendung im Pflanzenschutz je nach Art des gewünschten Effektes zwischen 0,01 und 2,0 kg Wirkstoff pro ha.

Bei der Saatgutbehandlung werden im allgemeinen Wirkstoffmengen von 1 bis 1000 g/100 kg Saatgut, vorzugsweise 1 bis 200 g/100 kg, insbesondere 5 bis 100 g/100 kg verwendet.

Bei der Anwendung im Material- bzw. Vorratsschutz richtet sich die Aufwandmenge an Wirkstoff nach der Art des Einsatzgebietes und des gewünschten Effekts. Übliche Aufwandmengen sind im Materialschutz beispielsweise 0,001 g bis 2 kg, vorzugsweise 0,005 g bis 1 kg Wirkstoff pro Kubikmeter behandelten Materials.

Die Verbindungen l können in die üblichen Formulierungen überführt werden, z.B. Lösungen, Emulsionen, Suspensionen, Stäube, Pulver, Pasten und Granulate. Die Anwendungsform richtet sich nach dem jeweiligen Verwendungszweck; sie soll in jedem Fall eine feine und gleichmäßige Verteilung der erfindungsgemäßen Verbindung gewährleisten.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln. Als Lösungsmittel / Hilfsstoffe kommen dafür im wesentlichen in Betracht:
- Wasser, aromatische Lösungsmittel (z.B. Solvesso Produkte, Xylol), Paraffine (z.B. Erdölfraktionen), Alkohole (z.B. Methanol, Butanol, Pentanol, Benzylalkohol), Ketone (z.B. Cyclohexanon, gamma-Butryolacton), Pyrrolidone (NMP, NOP), Acetate (Glykoldiacetat), Glykole, Dimethylfettsäureamide, Fettsäuren und Fettsäureester. Grundsätzlich können auch Lösungsmittelgemische verwendet werden,
- Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Lignin-Sulfitablaugen und Methylcellulose.

Als oberflächenaktive Stoffe kommen Alkali-, Erdalkali-, Ammoniumsalze von Ligninsulfonsäure, Naphthalinsulfonsäure, Phenolsulfonsäure, Dibutylnaphthalinsulfonsäure, Alkylarylsulfonate, Alkylsulfate, Alkylsulfonate, Fettalkoholsulfate, Fettsäuren und sulfatierte Fettalkoholglykolether zum Einsatz, ferner Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Tristerylphenylpolyglykolether, Alkylarylpolyetheralkohole, Alkohol- und Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Ligninsulfitablaugen und Methylcellulose in Betracht.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, lsophoron, stark polare Lösungsmittel, z.B. Dimethylsulfoxid, N-Methylpyrrolidon oder Wasser in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nussschalenmehl, Cellulosepulver und andere feste Trägerstoffe.

Die Formulierungen enthalten im allgemeinen zwischen 0,01 und 95 Gew.-%, vorzugsweise zwischen 0,1 und 90 Gew.-% des Wirkstoffs. Die Wirkstoffe werden dabei in einer Reinheit von 90% bis 100%, vorzugsweise 95% bis 100% (nach NMR-Spektrum) eingesetzt.

### Beispiele für Formulierungen sind: 1. Produkte zur Verdünnung in Wasser

A Wasserlösliche Konzentrate (SL)
   10 Gew.-Teile einer erfindungsgemäßen Verbindung werden in Wasser oder einem wasserlöslichen Lösungsmittel gelöst. Alternativ werden Netzmittel oder andere Hilfsmittel zugefügt. Bei der Verdünnung in Wasser löst sich der Wirkstoff.
B Dispergierbare Konzentrate (DC)
   20 Gew.-Teile einer erfindungsgemäßen Verbindung werden in Cyclohexanon unter Zusatz eines Dispergiermittels z.B. Polyvinylpyrrolidon gelöst. Bei Verdünnung in Wasser ergibt sich eine Dispersion.
C Emulgierbare Konzentrate (EC)
   15 Gew.-Teile einer erfindungsgemäßen Verbindung werden in Xylol unter Zusatz von Ca-Dodecylbenzolsulfonat und Ricinusölethoxylat (jeweils 5 %) gelöst. Bei der Verdünnung in Wasser ergibt sich eine Emulsion.
D Emulsionen (EW, EO)
   40 Gew.-Teile einer erfindungsgemäßen Verbindung werden in Xylol unter Zusatz von Ca-Dodecylbenzolsulfonat und Ricinusölethoxylat (jeweils 5 %) gelöst. Diese Mischung wird mittels einer Emulgiermaschine (Ultraturax) in Wasser eingebracht und zu einer homogenen Emulsion gebracht. Bei der Verdünnung in Wasser ergibt sich eine Emulsion.
E Suspensionen (SC, OD)
   20 Gew.-Teile einer erfindungsgemäßen Verbindung werden unter Zusatz von Dispergier- und Netzmitteln und Wasser oder einem organischen Lösungsmittel in einer Rührwerkskugelmühle zu einer feinen Wirkstoffsuspension zerkleinert. Bei der Verdünnung in Wasser ergibt sich eine stabile Suspension des Wirkstoffs.
F Wasserdispergierbare und wasserlösliche Granulate (WG, SG)
   50 Gew.-Teile einer erfindungsgemäßen Verbindung werden unter Zusatz von Dispergier- und Netzmitteln fein gemahlen und mittels technischer Geräte (z.B. Extrusion, Sprühturm, Wirbelschicht) als wasserdispergierbare oder wasserlösliche Granulate hergestellt. Bei der Verdünnung in Wasser ergibt sich eine stabile Dispersion oder Lösung des Wirkstoffs.
G Wasserdispergierbare und wasserlösliche Pulver (WP, SP)
   75 Gew.-Teile einer erfindungsgemäßen Verbindung werden unter Zusatz von Dispergier- und Netzmitteln sowie Kieselsäuregel in einer Rotor-Strator Mühle vermahlen. Bei der Verdünnung in Wasser ergibt sich eine stabile Dispersion oder Lösung des Wirkstoffs.

### 2. Produkte für die Direktapplikation

H Stäube (DP)
   5 Gew.Teile einer erfindungsgemäßen Verbindung werden fein gemahlen und mit 95 % feinteiligem Kaolin innig vermischt. Man erhält dadurch ein Stäubemittel.
I Granulate (GR, FG, GG, MG)
   0.5 Gew-Teile einer erfindungsgemäßen Verbindung werden fein gemahlen und mit 95.5 % Trägerstoffe verbunden. Gängige Verfahren sind dabei die Extrusion, die Sprühtrocknung oder die Wirbelschicht. Man erhält dadurch ein Granulat für die Direktapplikation.
J ULV- Lösungen (UL)
   10 Gew.-Teile einer erfindungsgemäßen Verbindung werden in einem organischen Lösungsmittel z.B. Xylol gelöst. Dadurch erhält man ein Produkt für die Direktapplikation.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Wässrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermitttel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Die Wirkstoffkonzentrationen in den anwendungsfertigen Zubereitungen können in größeren Bereichen variiert werden. Im allgemeinen liegen sie zwischen 0,0001 und 10%, vorzugsweise zwischen 0,01 und 1%.

Die Wirkstoffe können auch mit gutem Erfolg im Ultra-Low-Volume-Verfahren (ULV) verwendet werden, wobei es möglich ist, Formulierungen mit mehr als 95 Gew.-% Wirkstoff oder sogar den Wirkstoff ohne Zusätze auszubringen.

Zu den Wirkstoffen können Öle verschiedenen Typs, Netzmittel, Adjuvants, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln. Beim Vermischen der Verbindungen 1 bzw. der sie enthaltenden Mittel in der Anwendungsform als Fungizide mit anderen Fungiziden erhält man in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:
- Acylalanine wie Benalaxyl, Metalaxyl, Ofurace, Oxadixyl;
- Aminderivate wie Aldimorph, Dodine, Dodemorph, Fenpropimorph, Fenpropidin, Guazatine, Iminoctadine, Spiroxamin, Tridemorph;
- Anilinopyrimidine wie Pyrimethanil, Mepanipyrim oder Cyrodinyl;
- Antibiotika wie Cycloheximid, Griseofulvin, Kasugamycin, Natamycin, Polyoxin oder Streptomycin;
- Azole wie Bitertanol, Bromoconazol, Cyproconazol, Difenoconazole, Dinitroconazol, Enilconazol, Epoxiconazol, Fenbuconazol, Fluquiconazol, Flusilazol, Flutriafol, Hexaconazol, Imazalil, Metconazol, Myclobutanil, Penconazol, Propiconazol, Prochloraz, Prothioconazol, Tebuconazol, Triadimefon, Triadimenol, Triflumizol, Triticonazol;
- Dicarboximide wie lprodion, Myclozolin, Procymidon, Vinclozolin;
- Dithiocarbamate wie Ferbam, Nabam, Maneb, Mancozeb, Metam, Metiram, Propineb, Polycarbamat, Thiram, Ziram, Zineb;
- Heterocylische Verbindungen wie Anilazin, Benomyl, Boscalid, Carbendazim, Carboxin, Oxycarboxin, Cyazofamid, Dazomet, Dithianon, Famoxadon, Fenamidon, Fenarimol, Fuberidazol, Flutolanil, Furametpyr, lsoprothiolan, Mepronil, Nuarimol, Probenazol, Proquinazid, Pyrifenox, Pyroquilon, Quinoxyfen, Silthiofam, Thiabendazol, Thifluzamid, Thiophanat-methyl, Tiadinil, Tricyclazol, Triforine;
- Kupferfungizide wie Bordeaux Brühe, Kupferacetat, Kupferoxychlorid, basisches Kupfersulfat;
- Nitrophenylderivate, wie Binapacryl, Dinocap, Dinobuton, Nitrophthal-isopropyl;
- Phenylpyrrole wie Fenpiclonil oder Fludioxonil;
- Schwefel;
- Sonstige Fungizide wie Acibenzolar-S-methyl, Benthiavalicarb, Carpropamid, Chlorothalonil, Cyflufenamid, Cymoxanil, Dazomet, Diclomezin, Diclocymet, Diethofencarb, Edifenphos, Ethaboxam, Fenhexamid, Fentin-Acetat, Fenoxanil, Ferimzone, Fluazinam, Fosetyl, Fosetyl-Aluminium, lprovalicarb, Hexachlorbenzol, Metrafenon, Pencycuron, Propamocarb, Phthalid, Toloclofos-methyl, Quintozene, Zoxamid;
- Strobilurine wie Azoxystrobin, Dimoxystrobin, Fluoxastrobin, Kresoxim-methyl, Metominostrobin, Orysastrobin, Picoxystrobin, Pyraclostrobin oder Trifloxystrobin;
- Sulfensäurederivate wie Captafol, Captan, Dichlofluanid, Folpet, Tolylfluanid;
- Zimtsäureamide und Analoge wie Dimethomorph, Flumetover oder Flumorph.

### Synthesebeispiele

Die in den nachstehenden Synthesebeispielen wiedergegebenen Vorschriften wurden unter entsprechender Abwandlung der Ausgangsverbindungen zur Gewinnung weiterer Verbindungen I benutzt. Die so erhaltenen Verbindungen sind in der anschließenden Tabelle mit physikalischen Angaben aufgeführt.

### Beispiel 1: Herstellung von 2-Chlor-5-fluorphenylmalonsäurediethlester

Eine Suspension von Natriumhydrid (0,51 mol) in 140 ml 1,4-Dioxan wurde bei etwa 60°C während 2 Std. mit Malonsäurediethlester (0,49 mol) versetzt. Nach weiteren 10 min Rühren wurden 0,05 mol CuBr zugesetzt. Nach 15 min wurden 0,25 mol 2-Chlor-5-fluorbrombenzol in 10 ml Dioxan zugegeben. Die Reaktionsmischung wurde etwa 14 Std. bei 100°C gehalten, dann bei etwa 15°C langsam mit 35 ml 12N Salzsäure versetzt. Der Niederschlag wurde abfiltriert, das Filtrat mit Diethylether extrahiert. Nach Phasentrennung wurde die organische Phase getrocknet, dann vom Lösungsmittel befreit. Es blieben 42 g der Titelverbindung zurück.

### Beispiel 2: Herstellung von 5,7-Dihydroxy-6-(2-chlor-5-fluorphenylphenyl)-[1,2,4]-triazolo[1,5-a]-pyrimidin

Eine Mischung von 12 g 3-Amino-1,2,4-triazol, 0,17 mol des Esters aus Beispiel 1 und 50 ml Tributylamin wurde etwa sechs Std. bei 180°C gerührt. Bei etwa 70°C wurde eine Lösung von 21 g NaOH in 200 ml Wasser zugegeben und die Mischung weiter 30 min gerührt. Die organische Phase wurde abgetrennt und die wässrige Phase mit Diethylether extrahiert. Aus der wässrigen Phase fiel nach Ansäuern mit konz. Salzsäure das Produkt aus. Durch Filtration wurden 33 g der Titelverbindung erhalten.

### Beispiel 3: Herstellung von 5,7-Dichlor-6-(2-chlor-5-fluorphenyl)-[1,2,4]-triazolo[1,5-a]-pyrimidin

Eine Mischung von 30 g des Triazolopyrimidins aus Beispiel 2 und 50 ml POCl₃ wurde für acht Std. refluxiert, dabei destillierte etwas POCl₃ ab. Der Rückstand wurde in eine CH₂Cl₂-Wasser-Mischung gegeben, die organische Phase abgetrennt, gewaschen und getrocknet, dann vom Lösungsmittel befreit. Es wurden 27 g der Titelverbindung vom Fp. 137°C erhalten.

### Beispiel 4: Herstellung von 5-Chlor-6-(2-chlor 5-fluorphenyl)-7-but-2-ylamino-[1,2,4]-triazolo[1,5-a]pyrimidin [1-3]

Eine Lösung von 1,5 mmol des Produkts aus Bsp. 3 in 10 ml Dichlormethan wurde unter Rühren mit einer Lösung von 1,5 mmol 2-Butylamin, 1,5 mmol Triethylamin in 10 ml Dichlormethan versetzt. Die Reaktionsmischung wurde etwa 16 Std. bei 20 bis 25°C gerührt, dann mit verd. Salzsäure gewaschen. Die organische Phase wurde abgetrennt, getrocknet und vom Lösungsmittel befreit. Nach Chromatographie an Kieselgel wurden 35 mg der Titelverbindung vom Fp. 171 erhalten.

**Tabelle. 1 - Verbindungen der Formel 1**

| **Nr.** | **R¹** | **R²** | **L¹** | **L²** | **X** | **Phys. Daten (Fp. [°C]; HPLC/MS [Rₜ; m/z])** |
|---|---|---|---|---|---|---|
| I-1 | CH₂C(CH₃)=CH₂ | CH₂CH₃ | F | H | Cl | 133 |
| I-2 | -(CH₂)₂CH(CH₃)(CH₂)₂- | | F | H | Cl | 188 |
| I-3 | (±)CH(CH₃)-CH₂CH₃ | H | F | H | Cl | 171 |
| I-4 | (S) CH(CH₃)-CH₂CH₃ | H | F | H | Cl | 168 |
| I-5 | (R) CH(CH₃)-CH₂CH₃ | H | F | H | Cl | 168 |
| I-6 | (±) CH(CH₃)-CH(CH₃)₂ | H | F | H | Cl | 111 |
| I-7 | (S) CH(CH₃)-CH(CH₃)₂ | H | F | H | Cl | 109 |
| I-8 | (R) CH(CH₃)-CH(CH₃)₂ | H | F | H | Cl | 109 |
| I-9 | (±) CH(CH₃)-C(CH₃)₃ | H | F | H | Cl | 178 |
| I-10 | (S) CH(CH₃)-C(CH₃)₃ | H | F | H | Cl | 173 |
| I-11 | (R) CH(CH₃)-C(CH₃)₃ | H | F | H | Cl | 173 |
| I-12 | (±) CH(CH₃)-CF₃ | H | F | H | Cl | 155 |
| I-13 | (S) CH(CH₃)-CF₃ | H | F | H | Cl | 157 |
| I-14 | (R) CH(CH₃)-CF₃ | H | F | H | Cl | 157 |
| I-15 | CH₂CF₃ | H | F | H | Cl | 175 |
| I-16 | -(CH₂)₂-CH(CH₃)-(CH₂)₂- | | F | OCH₃ | Cl | Harz |
| I-17 | CH₂-C(=CH₂)-CH₃ | CH₂CH₃ | F | OCH₃ | Cl | 3,94 min; 410 (M+H)⁺ |
| I-18 | (±)CH(CH₃)C(CH₃)₃ | H | F | OCH₃ | Cl | 3,91 min; 412 (M+H)⁺ |
| I-19 | (±)CH(CH₃)CH(CH₃)₂ | H | F | OCH₃ | Cl | 3,71 min; 398 (M+H)⁺ |
| I-20 | (±)CH(CH3)CH2-CH3 | H | F | OCH₃ | Cl | 3,53 min; 384 (M+H)⁺ |
| I-21 | -CH(CH₃)-(CH₂)₃- | | F | OCH₃ | Cl | 3,62 min; 396 (M+H)⁺ |
| I-22 | -CH(CH₃)-(CH₂)₄- | | F | OCH₃ | Cl | 3,88 min; 410 (M+H)⁺ |
| I-23 | -CH₂-CH(CH₃)-(CH₂)₂- | | F | OCH₃ | Cl | 3,62 min; 396 (M+H)⁺ |
| I-24 | (R) CH(CH₃)-C(CH₃)₃ | H | F | OCH₃ | Cl | 3,91 min; 412 (M+H)⁺ |
| I-25 | (R) CH(CH₃)CH(CH₃)₂ | H | F | OCH₃ | Cl | 3,72 min; 398 (M+H)⁺ |
| I-26 | (±)CH(CH₃)-CF₃ | H | F | OCH₃ | Cl | 175-177 |
| I-27 | CH₂-CF₃ | H | F | OCH₃ | Cl | 191-193 |
| I-28 | CH₂-CF₃ | CH₃ | F | OCH₃ | Cl | 3,50 min; 424 (M+H)⁺ |
| I-29 | (S) CH(CH₃)-CF₃ | H | F | OCH₃ | Cl | 108-110 |
| I-30 | CH₂C(CH₃)=CH₂ | CH₂CH₃ | Cl | H | Cl | 128-130 |
| I-31 | -(CH₂)₂CH(CH₃)(CH₂)₂- | | Cl | H | Cl | 186-187 |
| I-32 | (±) CH(CH₃)C(CH₃)₃ | H | Cl | H | Cl | 157-158 |
| I-33 | (R)-CH(CH₃)-C(CH₃)₃ | H | Cl | H | Cl | 187-188 |
| I-34 | (±)-CH(CH₃)CH(CH₃)₂ | H | Cl | H | Cl | 142-144 |
| I-35 | (R)-CH(CH₃)CH(CH₃)₂ | H | Cl | H | Cl | 137-138 |
| I-36 | ±)-CH(CH₃)CH₂CH₃ | H | Cl | H | Cl | 109-110 |
| I-37 | (R)-CH(CH₃)CH₂CH₃ | H | Cl | H | Cl | 106-107 |
| I-38 | (±)-CH(CH₃)-CF₃ | H | Cl | | H Cl | 180-181 |
| I-39 | (S)-CH(CH₃)-CF₃ | H | Cl | | H Cl | 130-131 |
| I-40 | CH₂CF₃ | H | Cl | | H Cl | 185-190 |

HPLC/MS:
HPLC-Säule: RP-18 (Chromolith Speed ROD, Fa. Merck KgaA, Deutschland); Eluent: Acetonitril +0,1% Trifuoressigsäure (TFA)/Wasser + 0,1 % TFA in Gradienten 5:95 - 95:5 (5 min) /40°C.
MS: Quadrupol Elektrospray Ionisation, 80 V (positiv Modus)

### Beispiele für die Wirkung gegen Schadpilze

Die fungizide Wirkung der Verbindungen der Formel I ließ sich durch die folgenden Versuche zeigen:

Die Wirkstoffe wurden als eine Stammlösung aufbereitet mit 0,25 Gew.-% Wirkstoff in Aceton oder DMSO. Dieser Lösung wurde 1 Gew.-% Emulgator Uniperol® EL (Netzmittel mit Emulgier- und Dispergierwirkung auf der Basis ethoxylierter Alkylphenole) zugesetzt und entsprechend der gewünschten Konzentration mit Wasser verdünnt.

### Anwendungsbeispiel 1: Wirksamkeit gegen die Dürrfleckenkrankheit der Tomate verursacht durch Alternaria solani

Blätter von Topfpflanzen der Sorte "Goldene Prinzessin" wurden mit einer wässriger Suspension in der unten angegebenen Wirkstoffkonzen-tration bis zur Tropfnässe besprüht. Am folgenden Tag wurden die Blätter mit einer wässrigen Sporenaufschwemmung von *Alternaria solani* in 2 % Biomalzlösung mit einer Dichte von 0,17 x 10⁶ Sporen/ml infiziert. Anschließend wurden die Pflanzen in einer wasserdampf-gesättigten Kammer bei Temperaturen zwischen 20 und 22°C aufgestellt. Nach 5 Tagen hatte sich die Krankheit auf den unbehandelten, jedoch infizierten Kontrollpflanzen so stark entwickelt, dass der Befall visuell in % ermittelt werden konnte.

In diesem Test zeigten die mit 63 ppm der Verbindungen 1-3,1-13,1-15,1-23 bis 1-35, 1-38, bzw. 1-39 behandelten Pflanzen maximal 20 % Befall, während die unbehandelten Pflanzen zu 80 % befallen waren.

### Anwendungsbeispiel 2: Kurative Wirksamkeit gegen Weizenbraunrost verursacht durch Puccinia recondita

Blätter von in Töpfen gewachsenen Weizensämlingen der Sorte "Kanzler" wurden mit einer Sporensuspension des Braunrostes (*Puccinia recondita*) inokuliert. Danach wurden die Töpfe für 24 Stunden in eine Kammer mit hoher Luftfeuchtigkeit (90 bis 95 %) und 20 bis 22° C gestellt. Während dieser Zeit keimten die Sporen aus und die Keimschläuche drangen in das Blattgewebe ein. Die infizierten Pflanzen wurden am nächsten Tag mit einer wässriger Suspension in der unten angegebenen Wirkstoffkonzentration bis zur Tropfnässe besprüht. Die Suspension oder Emulsion wurde wie oben beschrieben hergestellt. Nach dem Antrocknen des Spritzbelages wurden die Versuchspflanzen im Gewächshaus bei Temperaturen zwischen 20 und 22° C und 65 bis 70 % relativer Luftfeuchte für 7 Tage kultiviert. Dann wurde das Ausmaß der Rostpilzentwicklung auf den Blättern ermittelt.

In diesem Test zeigten die mit 63 ppm der Verbindungen I-3, I-13, I-15, I-16, I-18 bis I-21, I-23 bis I-27, I-29, I-34 bis I-39, bzw. I-40 behandelten Pflanzen maximal 20 % Befall, während die unbehandelten Pflanzen zu 85 bis 90 % befallen waren.

## Patentansprüche

1. Triazolopyrimidine der Formel I 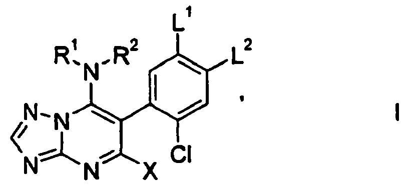 in der die Substituenten folgende Bedeutung haben:
R¹, R² unabhängig voneinander Wasserstoff, C₁-C₈-Alkyl, C₁-C₈-Halogenalkyl, C₃-C₈-Cycloalkyl, C₃-C₈-Halogencycloalkyl, C₂-C₈-Alkenyl, C₂-C₈-Halogenalkenyl, C₃-C₆-Cycloalkenyl, C₃-C₆-Halogencycloalkenyl, C₂-C₈-Alkinyl, C₂-C₈-Halogenalkinyl oder Phenyl, Naphthyl, oder ein fünf- oder sechsgliedriger gesättigter, partiell ungesättigter oder aromatischer Heterocyclus, enthaltend ein bis vier Heteroatome aus der Gruppe O, N oder S,
R¹ und R² können auch zusammen mit dem Stickstoffatom, an das sie gebunden sind, ein fünf- oder sechsgliedriges Heterocyclyl oder Heteroaryl bilden, welches über N gebunden ist und ein bis drei weitere Heteroatome aus der Gruppe O, N und S als Ringglied enthalten und/oder einen oder mehrere Substituenten aus der Gruppe Halogen, C₁-C₆-Alkyl, C₁-C₆-Halogenalkyl, C₂-C₆-Alkenyl, C₂-C₆-Halogenalkenyl, C₁-C₆-Alkoxy, C₁-C₆-Halogenalkoxy, C₃-C₆-Alkenyloxy, C₃-C₆-Halogenalkenyloxy, (exo)-C₁-C₆-Alkylen und Oxy-C₁-C₃-alkylenoxy tragen kann;
R¹ und/oder R² können eine bis vier gleiche oder verschiedene Gruppen R^{a} tragen:
R^{a} Halogen, Cyano, Nitro, Hydroxy, C₁-C₆-Alkyl, C₁-C₆-Halogenalkyl, C₁-C₆-Alkylcarbonyl, C₃-C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₂-C₈-Alkenyl, C₂-C₈-Halogenalkenyl, C₃-C₈-Cycloalkenyl, C₂-C₆-Alkenyloxy, C₃-C₆-Halogenalkenyloxy, C₂-C₆-Alkinyl, C₂-C₆-Halogenalkinyl, C₃-C₆-Alkinyloxy, C₃-C₆-Halogenalkinyloxy, C₃-C₆-Cycloalkoxy, C₃-C₆-Cycloalkenyloxy, Oxy-C₁-C₃-alkylenoxy, Phenyl, Naphthyl, fünf- bis zehngliedriger gesättigter, partiell ungesättigter oder aromatischer Heterocyclus, enthaltend ein bis vier Heteroatome aus der Gruppe O, N oder S, wobei diese aliphatischen, alicyclischen oder aromatischen Gruppen ihrerseits partiell oder vollständig halogeniert sein oder eine bis drei Gruppen R^{b} tragen können:
R^{b} Halogen, Cyano, Nitro, Hydroxy, Mercapto, Amino, Carboxyl, Aminocarbonyl, Aminothiocarbonyl, Alkyl, Haloalkyl, Alkenyl, Alkenyloxy, Alkinyloxy, Alkoxy, Halogenalkoxy, Alkylthio, Alkylamino, Dialkylamino, Formyl, Alkylcarbonyl, Alkylsulfonyl, Alkylsulfoxyl, Alkoxycarbonyl, Alkylcarbonyloxy, Alkylaminocarbonyl, Dialkylaminocarbonyl, Alkylaminothiocarbonyl, Dialkylaminothiocarbonyl, wobei die Alkylgruppen in diesen Resten 1 bis 6 Kohlenstoffatome enthalten und die genannten Alkenyl- oder Alkinylgruppen in diesen Resten 2 bis 8 Kohlenstoffatome enthalten;
und/oder einen bis drei der folgenden Reste:
Cycloalkyl, Cycloalkoxy, Heterocyclyl, Heterocyclyloxy, wobei die cyclischen Systeme 3 bis 10 Ringglieder enthalten; Aryl, Aryloxy, Arylthio, Aryl-C₁-C₆-alkoxy, Aryl-C₁-C₆-alkyl, Hetaryl, Hetaryloxy, Hetarylthio, wobei die Arylreste vorzugsweise 6 bis 10 Ringglieder, die Hetarylreste 5 oder 6 Ringglieder enthalten, wobei die cyclischen Systeme partiell oder vollständig halogeniert oder durch Alkyl- oder Haloalkylgruppen substituiert sein können.
L¹ Fluor, Chlor oder Brom;
L² Wasserstoff, C₁-C₄-Alkyl oder C₁-C₄-Alkoxy ; und
X Halogen, Cyano, C₁-C₄-Alkyl, C₁-C₄-Halogenalkyl, C₁-C₄-Alkoxy oder C₁-C₂-Halogenalkoxy.

2. Verbindungen der Formel l gemäß Anspruch 1, in der L² Wasserstoff bedeutet.

3. Verbindungen der Formel l gemäß Anspruch 1, in der L² Alkyl oder Alkoxy bedeutet.

4. Verbindungen der Formel I gemäß einem der Ansprüche 1 bis 3, in der L¹ Fluor bedeutet.

5. Verbindungen der Formel I gemäß einem der Ansprüche 1 bis 3, in der L¹ Chlor bedeutet.

6. Verbindungen der Formel I gemäß einem der Ansprüche 1 bis 5, in der R¹ nicht Wasserstoff bedeutet.

7. Verbindungen der Formel l gemäß einem der Ansprüche 1 bis 6; in der X Chlor bedeutet.

8. Verbindungen der Formel I.1: 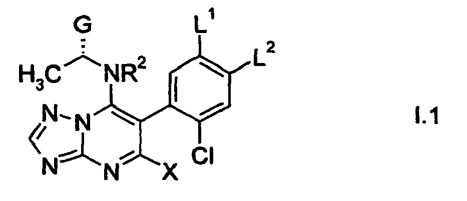 in der
G C₂-C₆-Alkyl, C₁-C₄-Alkoxymethyl oder C₃-C₈-Cycloalkyl;
R² Wasserstoff oder Methyl; und
X Chlor, Methyl, Cyano, Methoxy oder Ethoxy bedeuten
und L¹ und L² gemäß einem der Ansprüche 1 bis 5 definiert sind.

9. Verbindungen der Formel 1.2: 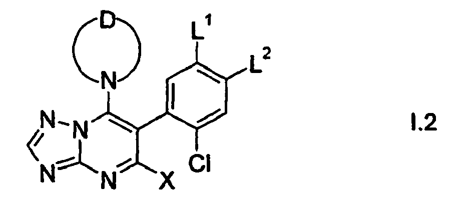 in der
D zusammen mit dem Stickstoffatom ein fünf- oder sechsgliedriges Heterocyclyl oder Heteroaryl bildet, welches über N gebunden ist und ein weiteres Heteroatom aus der Gruppe O, N und S als Ringglied enthalten und/oder einen oder mehrere Substituenten aus der Gruppe Halogen, C₁-C₄-Alkyl, C₁-C₄-Alkoxy und C₁-C₂-Halogenalkyl tragen kann;
X Chlor, Methyl, Cyano, Methoxy oder Ethoxy bedeuten.
und L¹ und L² gemäß einem der Ansprüche 1 bis 5 definiert sind.

10. Verbindungen der Formel I.3: 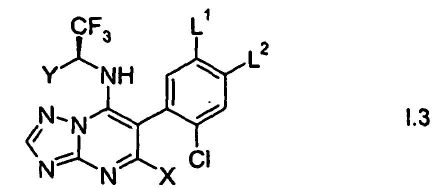 in der Y für Wasserstoff oder C₁-C₄-Alkyl;
X für Chlor, Methyl, Cyano, Methoxy oder Ethoxy steht und L¹ und L² gemäß einem der Ansprüche 1 bis 5 definiert sind.

11. Verfahren zur Herstellung der Verbindungen der Formel I gemäß einem der Ansprüche 1 bis 7, in der X für Halogen, Cyano, C₁-C₄-Alkyl, C₁-C₄-Alkoxy oder C₁-C₂-Halogenalkoxy steht, durch Umsetzung von 5-Aminotriazol der Formel II 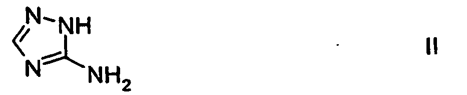 mit Phenymalonaten der Formel III 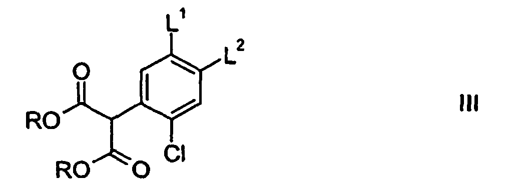 in der R für Alkyl steht, zu Dihydroxytriazolopyrimidinen der Formel IV, 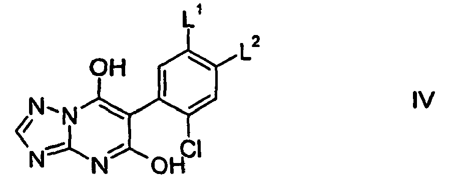 Halogenierung zu den Dihalogenverbindungen der Formel V, 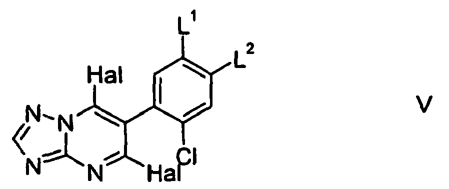 und Umsetzung von V mit Aminen der Formel VI 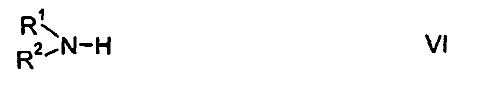 zu Verbindungen der Formel I, in der X für Halogen steht, gewünschtenfalls zu Herstellung von Verbindungen I, in denen X für Cyano, C₁-C₄-Alkoxy oder C₁-C₂-Halogenalkoxy steht, Umsetzung von Verbindungen I, in denen X Halogen bedeutet, mit Verbindungen der Formel VII,
M-X' VII
die je nach der einzuführenden Gruppe X' ein anorganisches Cyanid, ein Alkoxylat oder ein Halogenalkoxylat darstellen und in der M für ein Ammonium-, Tetraalkylammonium-, Alkali- oder Erdalkalimetallkation steht und gewünschtenfalls, zur Herstellung von Verbindungen der Formel l gemäß Anspruch 1, in der X für Alkyl steht, durch Umsetzung der Verbindungen l, in denen X für Halogen steht, mit Malonaten der Formel VIII, 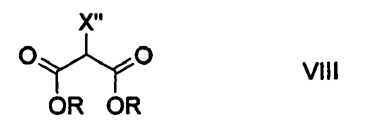 in der X" Wasserstoff oder C₁-C₃-Alkyl und R C₁-C₄-Alkyl bedeuten, zu Verbindungen der Formel IX 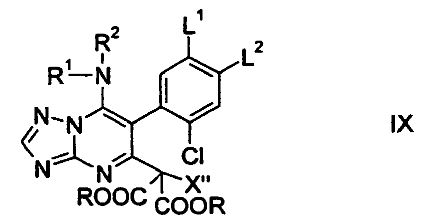 und Decarboxylierung zu Verbindungen 1, in denen X für Alkyl steht.

12. Verfahren zur Herstellung der Verbindungen der Formel I gemäß einem der Ansprüche 1 bis 6, in der X für C₁-C₄-Alkyl oder C₁-C₄-Halogenalkyl steht, durch Umsetzung von 5-Aminotriazol der Formel II gemäß Anspruch 11 mit Ketoestem der Formel.
IIIa, 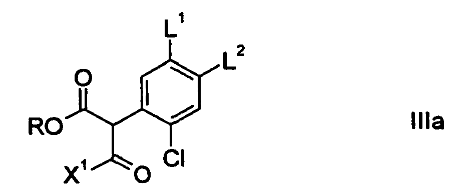 in der X' für C₁-C₄-Alkyl oder C₁-C₄-Halogenalkyl und R für C₁-C₄-Alkyl steht, zu 5-Alkyl-7-hydroxy-6-phenyltriazolopyrimidinen der Formel IVa 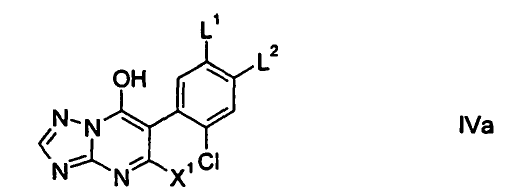 Halogenierung von IVa zu 7-Halogenotriazolopyrimidinen der Formel Va 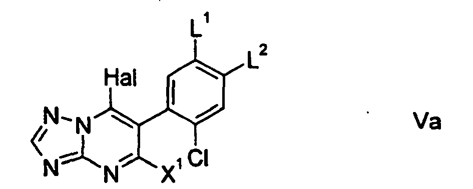 und Umsetzung von Va mit Aminen der Formel VI gemäß Anspruch 11 zu Verbindungen l.

13. Verbindungen der Formeln IV, IVa, V und Va gemäß Ansprüchen 11 und 12.

14. Fungizides Mittel, enthaltend einen festen oder flüssigen Träger und eine Verbindung der Formel l gemäß einem der Ansprüche 1 bis 7.

15. Saatgut, enthaltend 1 bis 1000 g einer Verbindung der Formel l gemäß einem der Ansprüche 1 bis 3 pro 100 kg.

16. Verfahren zur Bekämpfung von pflanzenpathogenen Schadpilzen, **dadurch gekennzeichnet, dass** man die Pilze, oder die vor Pilzbefall zu schützenden Materialien, Pflanzen, den Boden oder Saatgüter mit einer wirksamen Menge einer Verbindung der Formel 1 gemäß Ansprüchen 1 bis 7 behandelt.

## Claims

1. A triazolopyrimidine of the formula I 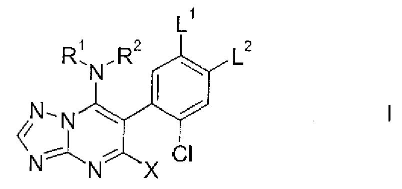 in which the substituents are as defined below:
R¹, R² independently of one another are hydrogen, C₁-C₈-alkyl, C₁-C₈-haloalkyl, C₃-C₈-cycloalkyl, C₃-C₈-halocycloalkyl, C₂-C₈-alkenyl, C₂-C₈-haloalkenyl, C₃-C₆-cycloalkenyl, C₃-C₆-halocycioalkenyl, C₂-C₈-alkynyl, C₂-C₈-haloalkynyl or phenyl, naphthyl, or a 5- or 6-membered saturated, partially unsaturated or aromatic heterocycle which contains one to four heteroatoms from the group consisting of O, N and S,
R¹ and R² together with the nitrogen atom to which they are attached may also form a 5- or 6-membered heterocyclyl or heteroaryl which is attached via N and may contain 1 to 3 further heteroatoms from the group consisting of O, N and S as ring members and/or may carry one or more substituents from the group consisting of halogen, C₁-C₆-alkyl, C₁-C₆-haloalkyl, C₂-C₆-alkenyl, C₂-C₆-haloalkenyl, C₁-C₆-alkoxy, C₁-C₆-haloalkoxy, C₃-C₆-alkenyloxy, C₃-C₆-haloalkenyloxy, (exo)-C₁-C₆-alkylene and oxy-C₁-C₃-alkyleneoxy;
R¹ and/or R² may carry one to four identical or different groups R^{a}:
R^{a} is halogen, cyano, nitro, hydroxyl, C₁-C₆-alkyl, C₁-C₆-haloalkyl, C₁-C₆-alkylcarbonyl, C₃-C₆-cycloalkyl, C₁-C₆-alkoxy, C₁-C₆-haloalkoxy, C₁-C₆-alkoxycarbonyl, C₁-C₆-alkylthio, C₁-C-₆-alkylamino, di-C₁-C₆-alkylamino, C₂-C₈-alkenyl, C₂-C₈-haloalkenyl, C₃-C₈-cycloalkenyl, C₂-C₆-alkenyloxy, C₃-C₆-haloalkenyloxy, C₂-C₆-alkynyl, C₂-C₆-haloalkynyl, C₃-C₆-alkynyloxy, C₃-C₆-haloalkynyloxy, C₃-C₆-cycloalkoxy, C₃-C₆-cycloalkenyloxy, oxy-C₁-C₃-alkyleneoxy, phenyl, naphthyl, a 5- to 10-membered saturated, partially unsaturated or aromatic heterocycle which contains one to four heteroatoms from the group consisting of O, N and S,
where these aliphatic, alicylic or aromatic groups for their part may be partially or fully halogenated or may carry one to three groups R^{b}:
R^{b} is halogen, cyano, nitro, hydroxyl, mercapto, amino, carboxyl, aminocarbonyl, aminothiocarbonyl, alkyl, haloalkyl, alkenyl, alkenyloxy, alkynyloxy, alkoxy, haloalkoxy, alkylthio, alkylamino, dialkylamino, formyl, alkylcarbonyl, alkylsulfonyl, alkylsulfoxyl, alkoxycarbonyl, alkylcarbonyloxy, alkylaminocarbonyl, dialkylaminocarbonyl, alkylaminothiocarbonyl, dialkylaminothiocarbonyl, where the alkyl groups in these radicals contain 1 to 6 carbon atoms and the alkenyl or alkynyl groups mentioned in these radicals contain 2 to 8 carbon atoms;
and/or one to three of the following radicals:
cycloalkyl, cycloalkoxy, heterocyclyl, heterocyclyloxy, where the cyclic systems contain 3 to 10 ring members; aryl, aryloxy, arylthio, aryl-C₁-C₆-alkoxy, aryl-C₁-C₆-alkyl, hetaryl, hetaryloxy, hetarylthio, where the aryl radicals preferably contain 6 to 10 ring members and the hetaryl radicals 5 or 6 ring members, where the cyclic systems may be partially or fully halogenated or substituted by alkyl or haloalkyl groups;
L¹ is fluorine, chlorine or bromine;
L² is hydrogen, C₁-C₄-alkyl or C₁-C₄-alkoxy; and
X is halogen, cyano, C₁-C₄-alkyl, C₁-C₄-haloalkyl, C₁-C₄-alkoxy or C₁-C₂-haloalkoxy.

2. The compound of the formula I according to claim 1 in which L² is hydrogen.

3. The compound of the formula I according to claim 1 in which L² is alkyl or alkoxy.

4. The compound of the formula I according to any of claims 1 to 3 in which L¹ is fluorine.

5. The compound of the formula I according to any of claims 1 to 3 in which L¹ is chlorine.

6. The compound of the formula I according to any of claims 1 to 5 in which R¹ is not hydrogen.

7. The compound of the formula I according to any of claims 1 to 6 in which X is chlorine.

8. A compound of the formula 1.1: 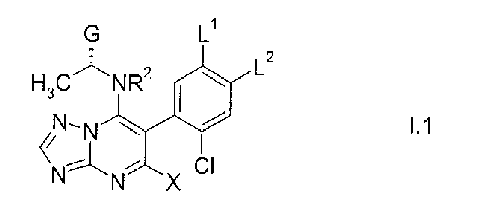 in which
G is C₂-C₆-alkyl, C₁-C₄-alkoxymethyl or C₃-C₆-cycloalkyl;
R² is hydrogen or methyl; and
X is chlorine, methyl, cyano, methoxy or ethoxy
and L¹ and L² are as defined in any of claims 1 to 5.

9. A compound of the formula 1.2: 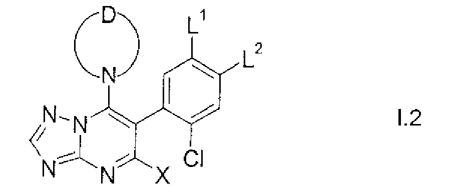 in which
D together with the nitrogen atom forms a 5- or 6-membered heterocyclyl or heteroaryl which is attached via N and may contain a further heteroatom from the group consisting of O, N and S as ring member and/or may carry one or more substituents from the group consisting of halogen, C₁-C₄-alkyl, C₁-C₄-alkoxy and C₁-C₂-haloalkyl;
X is chlorine, methyl, cyano, methoxy or ethoxy
and L¹ and L² are as defined in one of claims 1 to 5.

10. A compound of the formula 1.3: 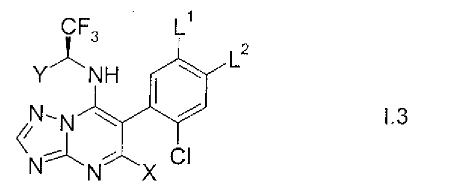 in which Y is hydrogen or C₁-C₄-alkyl;
X is chlorine, methyl, cyano, methoxy or ethoxy and L¹ and L² are as defined in any of claims 1 to 5.

11. A process for preparing a compound of the formula I according to either of claims 1 to 7, in which X is halogen, cyano, C₁-C₄-alkyl, C₁-C₄-alkoxy or C₁-C₂-haloalkoxy by reaction of 5-aminotraizole of the formula II 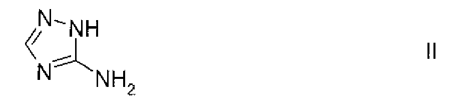 with phenylmalonates of the formula III 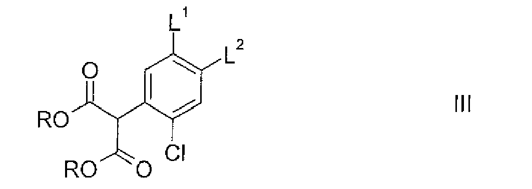 in which R is alkyl, to give dihydroxytriazolopyrimidines of the formula IV, 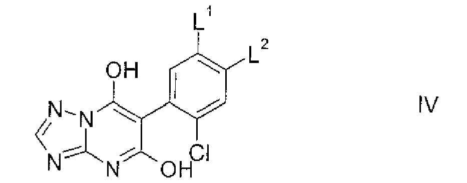 halogenation to give the dihalo compounds of the formula V, 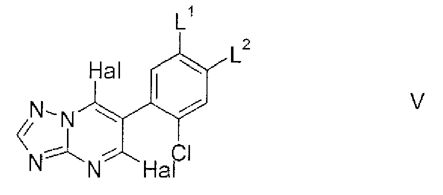 and reaction of V with amines of the formula VI 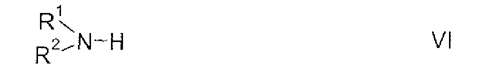 to give compounds of the formula I in which X is halogen, if desired, to prepare compounds I in which X is cyano, C₁-C₄-alkoxy or C₁-C₂-haloalkoxy, reaction of compounds I in which X is halogen with compounds of the formula VII,
M-X' VII
which, depending on the group X' to be introduced, are inorganic cyanides, alkoxides or haloalkoxides and in which M is an ammonium, tetraalkylammonium, alkali metal or alkaline earth metal cation, and, if desired, to prepare compounds of the formula I according to claim 1, in which X is alkyl, by reaction of the compounds I in which X is halogen with malonates of the formula VIII, 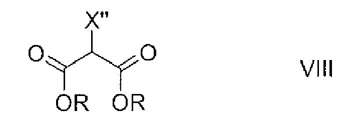 in which X" is hydrogen or C₁-C₃-alkyl and R is C₁-C₄-alkyl, to give compounds of the formula IX 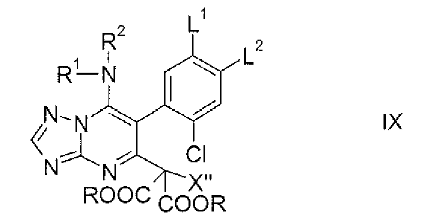 and decarboxylation to compounds I in which X is alkyl.

12. A process for preparing a compound of the formula I according to any of claims 1 to 6 in which X is C₁-C₄-alkyl or C₁-C₄-haloalkyl by reaction of 5-amirlotriazole of the formula 11 as set forth in claim 11 with keto esters of the formula IIIa, 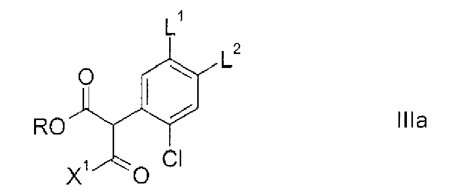 in which X¹ is C₁-C₄-alkyl or C₁-C₄-haloalkyl and R is C₁-C₄-alkyl, to give 5-alkyl-7-hydroxy-6-phenyltriazofopyrimidines of the formula IVa, 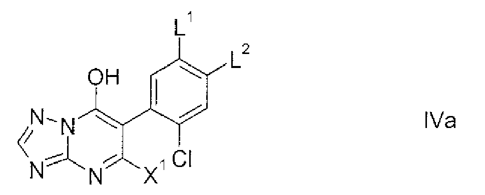 halogenation of IVa to give 7-halotriazolopyrimidines of the formula Va 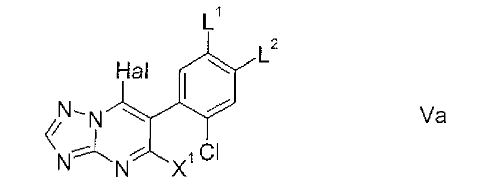 and reaction of Va with amines of the formula VI as set forth in claim 11 to give compounds I.

13. A compound of the formula IV, IVa, V or Va as set forth in claim 11 or 12.

14. A fungicidal composition, comprising a solid or liquid carrier and a compound of the formula I according to any of claims 1 to 7.

15. Seed, comprising 1 to 1000 g of a compound of the formula I according to any of claims 1 to 3 per 100 kg.

16. A method for controlling phytopathogenic harmful fungi, which method comprises treating the fungi or the materials, plants, the soil or seed to be protected against fungal attack with an effective amount of a compound of the formula I according to any of claims 1 to 7.

## Revendications

1. Triazolopyrimidines de la formule I : 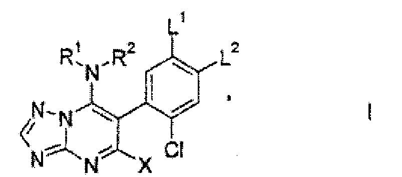 dans laquelle les substituants ont la signification suivante :
R¹, R² représentent indépendamment l'un de l'autre de l'hydrogène ou un groupe alkyle en C₁-C₈, halogénoalkyle en C₁-C₈, cycloalkyle en C₃-C₈, halogénocycloalkyle en C₃-C₈, alcényle en C₂-C₈, halogénoalcényle en C₂-C₈, cycloalcényle en C₃-C₆, halogénocycloalcényle en C₃-C₆, alcynyle en C₂-C₈, halogénoalcynyle en C₂-C₈ ou phényle, naphtyle, ou un hétérocycle pentagonal ou hexagonal saturé, partiellement insaturé ou aromatique, contenant un à quatre hétéroatomes du groupe O, N ou S,
R¹ et R² pouvant aussi former conjointement avec l'atome d'azote, sur lequel ils sont fixés, un hétéroaryle ou hétérocyclyle pentagonal ou hexagonal, qui est fixé par l'intermédiaire du N et peut contenir un à trois autres hétéroatomes du groupe O, N et S, comme chaînon du cycle, et/ou porter un ou plusieurs substituants du groupe halogène, alkyle en C₁-C₆, halogénoalkyle en C₁-C₆, alcényle en C₂-C₆, halogénoalcényle en C₂-C₆, alcoxy en C₁-C₆, halogénoalcoxy en C₁-C₆, alcényloxy en C₃-C₆, halogénoalcényloxy en C₃-C₆, (exo) -alkylène en C₁-C₆ et oxy-alkylèneoxy en C₁-C₃,
R¹ et/ou R² pouvant porter un à quatre groupes R^{a} identiques ou différents :
R^{a} représentant un halogène ou un groupe cyano, nitro, hydroxy, alkyle en C₁-C₆, halogénoalkyle en C₁-C₆, alkylcarbonyle en C₁-C₆, cycloalkyle en C₃-C₆, alcoxy en C₁-C₆, halogénoalcoxy en C₁-C₆, alcoxycarbonyle en C₁-C₆, alkylthio en C₁-C₆, alkylamino en C₁-C₆, di-C₁-C₆-alkylamino, alcényle en C₂-C₈, halogéno-alcényle en C₂-C₈, cycloalcényle en C₃-C₈, alcényloxy en C₂-C₆, halogénoalcényloxy en C₃-C₆, alcynyle en C₂-C₆, halogénoalcynyle en C₂-C₆, alcynyloxy en C₃-C₆, halogénoalcynyloxy en C₃-C₆, cycloalcoxy en C₃-C₆, cycloalcényloxy en C₃-C₆, oxy-alkylèneoxy en C₁-C₃, phényle, naphtyle, un hétérocycle pentagonal à hexagonal saturé, partiellement insaturé ou aromatique, contenant un à quatre hétéroatomes du groupe O, N ou S,
ces groupes aliphatiques, alicycliques ou aromatiques pouvant à leur tour être partiellement ou totalement halogénés ou porter un à trois groupes R^{b},
R^{b} représentant un halogène ou un groupe cyano, nitro, hydroxy, mercapto, amino, carboxyle, aminocarbonyle, aminothiocarbonyle, alkyle, halogénoalkyle, alcényle, alcényloxy, alcynyloxy, alcoxy, halogénoalcoxy, alkylthio, alkylamino, dialkylamino, formyle, alkylcarbonyle, alkylsulfonyle, alkylsulfoxyle, alcoxycarbonyle, alkylcarbonyloxy,
alkylaminocarbonyle, dialkylaminocarbonyle, alkylaminothiocarbonyle, dialkylaminothiocarbonyle, les groupes alkyle dans ces radicaux contenant 1 à 6 atomes de carbone et les groupes alcényle ou alcynyle cités contenant dans ces radicaux 2 à 8 atomes de carbone,
et/ou un à trois des radicaux suivants :
cycloalkyle, cycloalcoxy, hétérocyclyle, hétérocyclyloxy, les systèmes cycliques contenant 3 à 10 chaînons, aryle, aryloxy, arylthio, aryl-C₁-C₆-alcoxy, aryl-C₁-C₆-alkyle, hétaryle, hétaryloxy, hétarylthio, les radicaux aryle contenant de préférence 6 à 10 chaînons, les radicaux hétaryle 5 ou 6 chaînons, les systèmes cycliques pouvant être partiellement ou totalement halogénés ou substitués par des groupes alkyle ou halogénoalkyle,
L¹ représente du fluor, du chlore ou du brome,
L² représente de l'hydrogène ou un groupe alkyle en C₁-C₄ ou alcoxy en C₁-C₄, et
X représente de l'halogène ou un groupe cyano, alkyle en C₁-C₄, halogénoalkyle en C₁-C₄, alcoxy en C₁-C₄ ou halogénoalcoxy en C₁-C₂.

2. Composés de la formule I suivant la revendication 1, dans laquelle L² représente de l'hydrogène.

3. Composés de la formule I suivant la revendication 1, dans laquelle L² représente un groupe alkyle ou alcoxy.

4. Composés de la formule I suivant l'une des revendications 1 à 3, dans laquelle L¹ représente du fluor.

5. Composés de la formule I suivant l'une des revendications 1 à 3, dans laquelle L¹ représente du chlore.

6. Composés de la formule I suivant l'une des revendications 1 à 5, dans laquelle R¹ ne représente pas de l'hydrogène.

7. Composés de la formule I suivant l'une des revendications 1 à 6, dans laquelle X représente du chlore.

8. Composés de la formule générale I.1 : 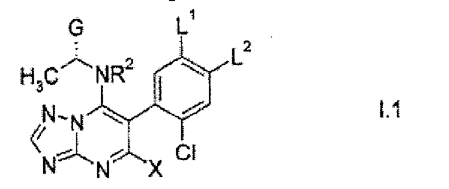 dans laquelle
G représente un groupe alkyle en C₂-C₆, C₁-C₄-alcoxyméthyle ou cycloalkyle en C₃-C₆,
R² représente de l'hydrogène ou du méthyle, et
X représente du Chlorure, du méthyle, du cyano, du méthoxy ou de l'éthoxy, et
L¹ et L² sont définis conformément à une des revendications 1 à 5.

9. Composés de la formule I.2 : 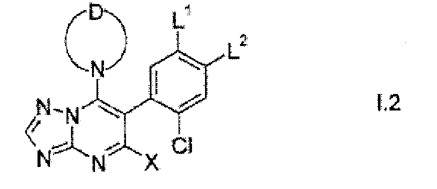 dans laquelle
D forme, conjointement à l'atome d'azote, un groupe hétéroaryle ou hétérocyclyle pentagonal ou hexagonal, qui est fixé par l'intermédiaire du N et peut contenir un autre hétéroatome du groupe O, N et S comme chaînon du cycle et/ou porter un ou plusieurs substituants du groupe halogène, alkyle en C₁-C₄, alcoxy en C₁-C₄ et halogénoalkyle en C₁-C₂,
X représente du chlore, du méthyle, du cyano, du méthoxy ou de l'éthoxy,
et L¹ et L² sont définis conformément à une des revendications 1 à 5.

10. Composés de la formule I.3 : 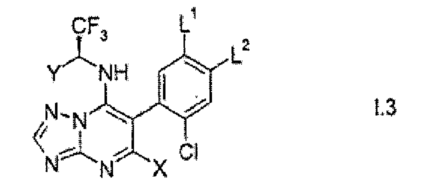 dans laquelle Y représente de l'hydrogène ou un groupe alkyle en C₁-C₄,
X représente du chlore, du méthyle, du cyano, du méthoxy ou de l'éthoxy, et
L¹ et L² sont définis conformément à l'une des revendications 1 à 5.

11. Procédé de préparation des composés de la formule I suivant l'une des revendications 1 à 7, dans laquelle X représente de l'halogène ou un groupe cyano, alkyle en C₁-C₄, alcoxy en C₁-C₄ ou halogénoalcoxy en C₁-C₂, par réaction de 5-aminotriazole de la formule II : 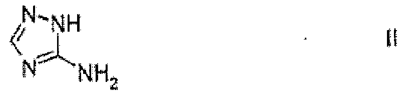 avec des phénylmalonates de la formule III : 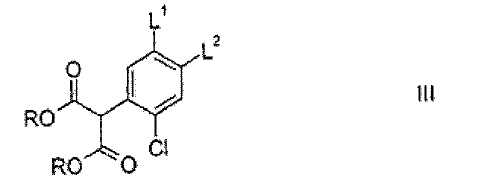 dans laquelle R représente un groupe alkyle, ce qui donne des dihydroxytriazolopyrimidines de la formule IV : 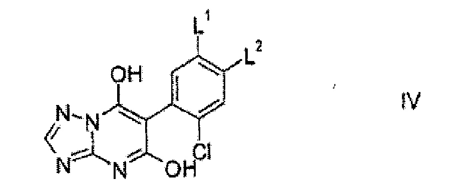 halogénation en les composés dihalogénés de la formule V : 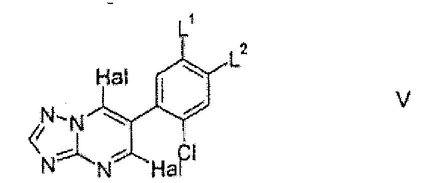 et réaction de V avec des amines de la formule VI : 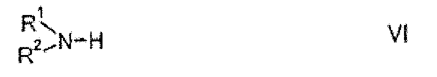 pour former des composés de la formule I, dans laquelle X représente de l'halogène, éventuellement pour la préparation de composés I, dans lesquels X représente un groupe cyano, alcoxy en C₁-C₄ ou halogénoalcoxy en C₁-C₂, par réaction de composés I, dans lesquels X représente de l'halogène, avec des composés de la formule VII :
M-X' VII
qui, selon le groupe X' à introduire, représentent un cyanure inorganique, un alcoolate ou un alcoolate halogéné et dans lesquels M représente un cation ammonium, tétraalkylammonium, alcalin ou alcalino-terreux et éventuellement, pour la préparation de composés de la formule I selon la revendication 1 dans lesquels X représente un groupe alkyle, par réaction des composés I, dans lesquels X représente de l'halogène, avec des malonates de la formule VIII : 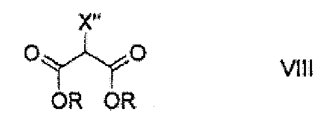 dans laquelle X" représente de l'hydrogène ou un groupe alkyle en C₁-C₃ et R représente un groupe alkyle en C₁-C₄, pour former des composés de la formule IX : 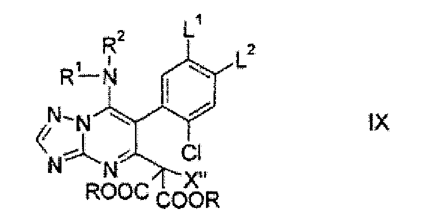 et décarboxylation en composés I, dans lesquels X représente un groupe alkyle.

12. Procédé de préparation des composés de la formule I suivant l'une des revendications 1 à 6, dans laquelle X représente un groupe alkyle en C₁-C₄ ou halogénoalkyle en C₁-C₄, par réaction de 5-aminotriazole de la formule II suivant la revendication 11 avec des cétoesters de la formule IIIa : 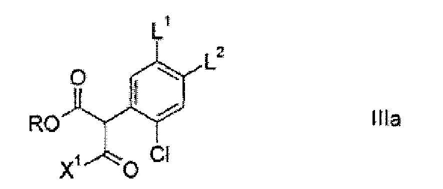 dans laquelle X¹ représente un groupe alkyle en C₁-C₄ ou halogénoalkyle en C₁-C₄ et R un groupe alkyle en C₁-C₄, pour former des 5-alkyl-7-hydroxy-6-phényltriazolopyrimidines de la formule IVa : 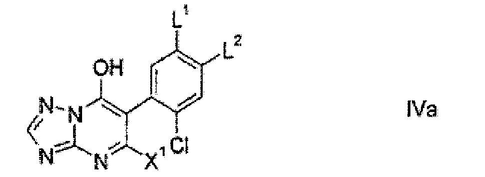 halogénation de IVa en 7-halogénotriazolopyrimidines de la formule Va : 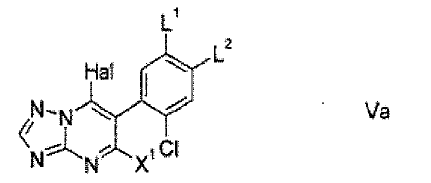 et réaction de Va avec des amines de la formule VI suivant la revendication 11, pour former des composés I.

13. Composés des formules IV, IVa, V et Va suivant les revendications 11 et 12.

14. Produits fongicides, contenant un support solide ou liquide et un composé de la formule I suivant l'une des revendications 1 à 7.

15. Semences, contenant 1 à 1000 g d'un composé de la formule I suivant l'une des revendications 1 à 3, par 100 kg.

16. Procédé de lutte contre des champignons nuisibles phytopathogènes, **caractérisé en ce qu'**on traite les champignons ou les matériels, plantes, sols ou semences à protéger d'une infestation par des champignons avec une quantité efficace d'un composé de la formule I suivant les revendications 1 à 7.
